# EUROPEAN PATENT SPECIFICATION

(11) **EP 2 358 422 B1**
(45) Date of publication and mention of the grant of the patent: **11.12.2019**
(21) Application number: 09793407.9
(22) Date of filing: 15.12.2009
(51) Int. Cl.: A61M 25/00, A61B 18/00, A61B 18/22, A61B 1/00, A61B 1/005, A61M 25/01

(54) **STEERABLE LASER-ENERGY DELIVERY DEVICE**
STEUERBARE VORRICHTUNG ZUR ABGABE VON LASERENERGIE
DISPOSITIF DE DISTRIBUTION D'ÉNERGIE LASER POUVANT ÊTRE DIRIGÉ

(30) Priority: 19.12.2008 US 340350; 24.06.2009 US 490827; 15.07.2009 US 503351
(43) Date of publication of application: 24.08.2011
(73) Proprietor: Boston Scientific Scimed, Inc., Maple Grove, MN 55311-1566 (US)
(72) Inventor: ZERFAS, Jeffrey, W., Bloomington Indiana 47401 (US); OSTROVSKY, Isaac, Wellesley Massachusetts 02481 (US)
(74) Representative: Pfenning, Meinig & Partner mbB
(86) International application number: PCT/US2009/068009
(87) International publication number: WO 2010/080393

(56) References cited:
- EP-A1- 0 900 547
- DE-A1- 4 309 805
- US-A- 6 042 581
- US-A1- 2006 142 732
- US-B1- 6 530 913

## Description

### TECHNICAL FIELD

The invention generally relates to a steerable medical device, and more particularly to a steerable laser-energy delivery device for delivering laser energy to a target position in a body of a patient.

### BACKGROUND INFORMATION

A variety of known endoscope type medical devices can be used during a medical procedure related to, for example, a ureteroscopy or colonoscopy. Some of these known endoscope types include and/or can be used with a laser-energy-delivery device configured for treatment of a target area (e.g., a tumor, a lesion, a stricture). The laser-energy-delivery device can include an optical fiber through which laser energy is delivered to the target area from a laser energy source. Laser energy from the laser energy source can be emitted into a proximal end (also can be referred to an entry end) of the optical fiber and propagated along the optical fiber until the laser energy is delivered to the target area out of a distal end of the optical fiber.

Laser energy that is not completely delivered into the proximal end of the optical fiber (can be referred to as stray laser energy or leaked laser energy) can adversely affect the mechanical properties and/or optical properties of the laser-energy-delivery system. For example, the stray laser energy can result in inefficient delivery of laser energy and/or damage to the laser-energy-delivery system. In some cases, an optical fiber can be susceptible to burning and/or breaking during operation when stray laser energy enters into and weakens a coating around the optical fiber. The stray laser energy can enter into, for example, a cladding layer of the optical fiber and can overfill the cladding in an undesirable fashion (e.g., a damaging fashion) when the optical fiber is bent during operation. The stray laser energy can be caused by misalignment of an output focal spot of the laser energy source with the proximal end of the optical fiber because of, for example, improper maintenance of the laser energy source or focal spot drift.

Although known coupling components (e.g., tapered coupling components) have been designed to deal with stray laser energy, these known coupling components can lack stability, can increase the effective numerical aperture (NA) of guided light which can lead to premature failure of a laser fiber when bent, redirect laser energy inefficiently, are relatively expensive to manufacture, and/or require relatively large heat sinks. Thus, a need exists for a coupling component that can increase the longevity of a laser-energy-delivery system, increase laser energy transmission efficiency, and/or reduce heat sink requirements.

In some medical procedures, such as those to treat conditions in the upper urinary tract of a patient, medical instruments must be inserted into the body of the patient and positioned at a target site within the patient's body. In some procedures, an endoscope, such as a cystoscope, is first introduced into the bladder of the patient. A guidewire or another medical instrument then is introduced into the patient's body through the cystoscope. The guidewire is passed through a working channel of the cystoscope until the distal or insertion end of the guidewire exits the distal end of the cystoscope and enters the bladder of the patient. The advancing distal end of the guidewire must then somehow be directed to the target location, such as to and through the entrance of the patient's ureter. Directing the guidewire into the patient's ureter with known techniques and tools often proves difficult.

In some medical procedures, it may be desirable to maneuver the distal end of an optical fiber of a laser-energy delivery device to a target area within a patient's body. The ability to bend, angle or curve a distal portion of the optical fiber may be desirable, but can sometimes result in damage to the optical fiber and/or stray laser energy can enter into and weaken a coating around the optical fiber. To help overcome issues of breakage or stray laser energy, some known optical fibers used in laser delivery devices have a large diameter fiber core (e.g., 550 microns) to provide sufficient stiffness to control the placement of the fiber tip. Such large diameter fiber cores may also be needed to support laser power at higher wattages, such as, for example, 100 Watts or greater and/or to add strength to the fiber /cap interface of the optical fiber. Unfortunately, such large fibers are not ideal for use in certain areas of the body and are typically too stiff to allow for the optical fiber to bend or be easily maneuvered within the patient's body. Side fire laser delivery systems are known, and can be used to direct laser energy at various angles relative to the laser fiber axis, but these too can have limitations on the maneuverability of the optical fiber for similar reasons as noted above. For example, document US 6,530,913 B1 discloses a steerable laser catheter wherein a sheath within which optical fibers (laser purpose) are disposed may be made from a shape memory material such as nitinol. Said sheath may be designed to deflect at a certain point. Deflection is achieved by pull-cable wires.

### SUMMARY OF THE INVENTION

The invention is defined in claim 1. Further aspects and preferred embodiments are defined in the dependent claims. Aspects and examples which do not fall within the scope of the appended claims do not form part of the invention as claimed and are provided for illustrative purposes only.

In one embodiment, an apparatus includes an optical fiber that includes a fiber core with a substantially constant outer diameter of less than or equal to 250 microns extending to a distal end of the optical fiber. The optical fiber is also configured to deliver laser energy up to at least 100 watts to a target area within a patient. The optical fiber is sufficiently flexible such that the optical fiber can be moved between a first configuration in which a distal end portion of the optical fiber is substantially linear and defines a longitudinal axis and a second configuration in which the distal end portion of the optical fiber is moved off its longitudinal axis. The apparatus also includes a steering mechanism coupled to the optical fiber. The steering mechanism is configured to move the optical fiber between its first configuration and its second configuration.

It is an object of the invention to controllably direct an optical fiber for use in a laser-energy delivery device to a target position within a body of a patient, such as, for example, a ureter a bladder a prostate or other area of the patient. A steerable medical device is described herein that can be used to direct an optical fiber or other instrument to a desired target location. The device can be used with an endoscope (whether rigid, semi-rigid, or flexible) or with some other tool, particularly by passing the steerable medical device through a working channel of the endoscope or other tool. Whether or not used through the working channel of an endoscope or other tool, the steerable medical device achieves easily and inexpensively the desired enhanced distal directability of an optical fiber used to deliver laser energy to a target location in a patient. When coupled to and passed through the working channel of an endoscope or other tool, a steerable medical device according to the invention can allow, with one-handed proximal operation, the distal manipulation required to controllably direct the distal end of the optical fiber or other instrument to the desired target location within a patient's body.

### BRIEF DESCRIPTION OF THE DRAWINGS

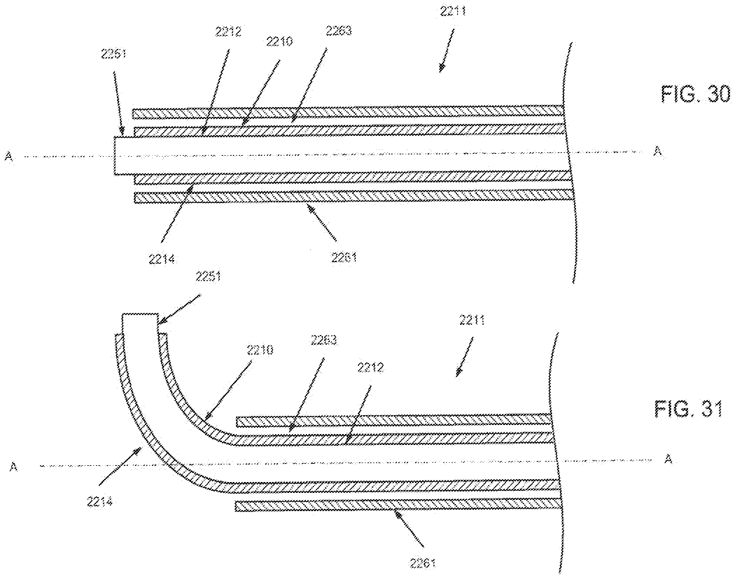

These and other features and advantages of the present invention will become better understood by reference to the following detailed description when considered in conjunction with the accompanying drawings. The drawings are for illustrative purposes only and are not necessarily to scale. Generally, emphasis is placed on conveying certain concepts and aspects according to the invention, therefore the actual dimensions of embodiments of the present invention, and their proportions to other medical instruments, may vary from the drawings. An embodiment of the invention as claimed is illustrated best in FIGS. 30 and 31.
FIG. 1 is a schematic illustration of a steerable medical device according to an example.
FIG. 2 is a cross-section of the steerable medical device of FIG. 1 taken along line A-A.
FIGS. 3 and 4 are side views of a steerable medical device according to an example.
FIG. 5 is a top view of a portion of the steerable medical device of FIG. 3.
FIG. 6 is a cross-section of the portion of the steerable medical device of FIG. 5 taken along line C-C.
FIG. 7 is a cross-section of a portion of the steerable medical device of FIG. 3 taken along line B-B.
FIG. 8 is an end view of the steerable medical device of FIG. 3.
FIG. 9 is an embodiment of a portion of a steerable medical device according to an example.
FIG. 10 is an embodiment of a portion of a steerable medical device according to an example.
FIGS. 11 - 13 are side views of the steerable medical device of FIG. 3 attached to an endoscope in a first, second, and third configuration, respectively.
FIG. 14 is a side view of the endoscope of FIGS. 11 - 13 with the steerable medical device removed.
FIG. 15 is a schematic diagram of a side cross-sectional view of a connector portion of a laser-energy delivery device, according to an example.
FIG. 16A is a schematic diagram of a side cross-sectional view of a connector portion of a laser-energy delivery device, according to an example.
FIG. 16B is a schematic diagram of the proximal end of the connector portion shown in FIG. 16A, according to an example.
FIG. 17 is a flow chart that illustrates a method for producing a connector portion of a laser-energy delivery device, according to an example.
FIG. 18 is a schematic diagram that illustrates a side cross-sectional view of a doped silica capillary that has a receiving portion, according to an example.
FIG. 19 is a schematic diagram that illustrates at least a portion of a laser-energy delivery device disposed within a housing assembly, according to an example.
FIG. 20 is a schematic diagram of a side cross-sectional view of a capillary holder, according to an example.
FIG. 21 is a schematic diagram of a side cross-sectional view of an alignment assembly, according to an example.
FIG. 22A is a schematic diagram of a side cross-sectional view of a grip assembly 895, according to an example.
FIG. 22B is a schematic diagram of an enlarged view of the side cross-sectional view of the grip assembly shown in FIG. 22A, according to an example.
FIG. 23 is schematic illustration of a steerable laser-energy delivery device according to an example, shown in a first configuration.
FIG. 24 is a side cross-sectional view of a distal portion of the steerable laser-energy delivery device of FIG. 23, shown in the first configuration.
FIG. 25 is a side view of a distal portion of the steerable medical device, shown in a second configuration.
FIG. 26 is a perspective view of a distal end portion of the steerable laser-energy delivery device of FIGS. 23-25 and an endoscope.
FIG. 27 is a cross-sectional view of a portion of an optical fiber according to an example.
FIG. 28 is a cross-sectional view of a portion of the optical fiber of FIG. 27 shown with an outer layer removed.
FIG. 29 is a flowchart illustrating a method according to an example.
FIG. 30 is a side view of a distal portion of a steerable laser-energy delivery device according to an embodiment of the invention, shown in a first configuration.
FIG. 31 is a side view of the distal portion of the steerable laser-energy delivery device of FIG. 30, shown in a second configuration.

### DESCRIPTION

Apparatuses and methods are described herein for use in the treatment of various conditions and in various locations within a patient's body, such as, for example, within a ureter, a bladder, a prostate or other area of the patient. In some examples, a steerable medical device is described that can controllably direct a medical tool or other device to a target location within a patient. The medical device to be directed to a target location can be, for example, a guidewire, a stone retrieval basket, a biopsy tool, a laser fiber, a small catheter or other tool. The steerable medical device can be used with an endoscope (whether rigid, semi-rigid, or flexible) or with some other tool, particularly by passing the steerable medical device through a working channel of the endoscope or other tool.

In some embodiments, a laser-energy delivery device is described. In some embodiments, a laser-energy delivery device can include a connector portion configured to receive laser energy emitted from a laser energy source. In some embodiments, a steerable medical device can include, or be used in conjunction with, such a laser-energy delivery device. A steerable medical device can alternatively include other embodiments of a laser-energy delivery device and/or other embodiments of an optical fiber as described in more detail below. For example, in some embodiments, an optical fiber can be provided that is sufficiently flexible to allow the optical fiber to be bent, curved or angled away from its longitudinal axis. Such an optical fiber can be maneuvered within a patient's body using a steering mechanism.

It is noted that, as used in this written description and the appended claims, the singular forms "a," "an" and "the" include plural referents unless the context clearly dictates otherwise. Thus, for example, the term "a wavelength" is intended to mean a single wavelength or a combination of wavelengths. Furthermore, the words "proximal" and "distal" refer to direction closer to and away from, respectively, an operator (e.g., a medical practitioner, a nurse, a technician, etc.) who would insert the medical device into the patient. Thus, for example, a laser energy deliver device end inserted inside a patient's body would be the distal end of the laser energy deliver device, while the laser energy deliver device end outside a patient's body would be the proximal end of the laser energy deliver device.

As described above, apparatuses for directing the introduction and insertion of another medical instrument (such as a guidewire, stone retrieval basket, biopsy tool, laser fiber, small catheter, etc.) to a target location in a body of a patient are described herein, as are related methods. These apparatuses can be used through the working channel of an endoscope (whether rigid, semi-rigid, or flexible) or other tool. In some examples, a steerable medical device is configured to be removably coupled to a rigid endoscope, some other type of endoscope (e.g., semi-rigid or flexible), or some other type of tool having a working channel and typically having some imaging capability as an endoscope usually does. A portion of the steerable medical device can be inserted into the body of the patient via the endoscope or else it can be inserted directly into the patient's body, and in any event the steerable medical device can be used to controllably introduce and direct a guidewire, or other medical instrument, into the body of the patient. The steerable medical device is adapted to direct the advancing end of the guidewire or other instrument to a target location in the body of the patient. The steerable medical device can then be uncoupled from the endoscope or other tool and removed from the patient's body while leaving the guidewire or other medical instrument in the body of the patient.

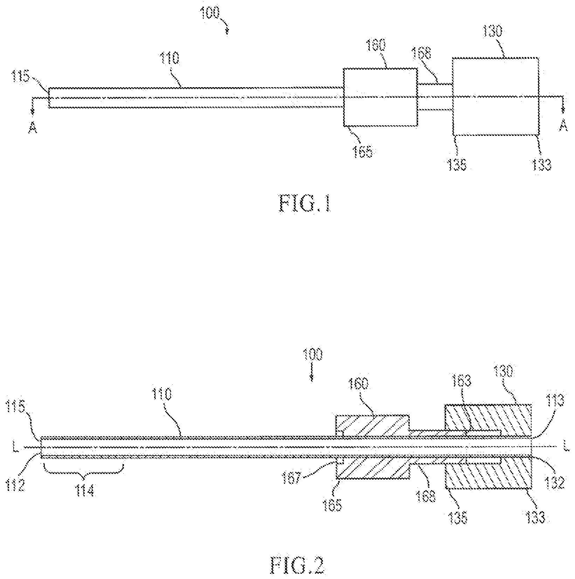

In one example, as schematically illustrated in FIGS. 1 and 2, a steerable medical device (also referred to herein as "device") 100 includes an elongated member 110, a steering mechanism 130, and an attachment member 160. At least a portion of the device 100 can be adapted to be received by (or inserted into) a working channel of an endoscope (whether rigid, semi-rigid, or flexible) or other such tool or medical device. For example, at least a portion of the elongated member 110 can be adapted to be received by the working channel of a rigid endoscope such as a cystoscope or a laparoscope. Although the steerable medical device 100 is capable of being used on its own without passing through the working channel of some type of endoscope or other tool, it can be particularly useful when used through the working channel of an endoscope or other tool and perhaps most useful when used through the working channel of a rigid or semi-rigid endoscope.

The elongated member 110 can be tubular and includes a proximal end 113 and a distal end 115 and defines a lumen 112 extending from the proximal end to the distal end. The elongated member 110 includes a deflectable portion 114. The entirety of the elongated member 110 extends along a longitudinal axis L when the deflectable portion 114 is straight or substantially straight. The deflectable portion 114 can be deflected off of the axis L. The deflectable portion 114 includes the distal end 115 of the elongated member 110.

The steering mechanism 130 is adapted to control deflection of the deflectable portion 114 of the elongated member 110. The steering mechanism 130 is disposed at or over the proximal end 113 of the elongated member 110. The steering mechanism 130 includes a proximal end 133 and a distal end 135. The steering mechanism 130 also defines an opening or lumen 132. In some examples, as illustrated in FIG. 2, the lumen 132 of the steering mechanism 130 receives at least a portion of the elongated member 110 including the proximal end 113.

In some examples, the steering mechanism 130 is coupled to the elongated member 110. For example, as illustrated in FIG. 2, the proximal end 133 of the steering mechanism 130 is fixedly coupled (by, for example, an adhesive, an interference fit, or in some other manner) to the proximal end 113 of the elongated member 110. Because the steering mechanism 130 and the elongated member 110 are fixedly coupled, rotation of the steering mechanism in one direction (such as clockwise about the axis L) correspondingly rotates the elongated member in the same direction. Furthermore, because the steering mechanism 130 and elongated member 110 are fixedly coupled, movement of the steering mechanism 130 in a longitudinal direction (meaning in a distal or proximal direction, such as along the axis L) correspondingly moves the elongated member 110 in the same longitudinal direction.

The elongated member 110 is also referred to herein as the tubular member 110, although the shape of the elongated member 110 does not have to be cylindrical. It can have any of a variety of cross-sectional shapes instead of circular, but a circular or substantially circular cross-sectional shape for the elongated member 110 is acceptable.

The attachment member 160 is adapted to removably couple the steerable medical device 100 to an endoscope (whether rigid, semi-rigid, or flexible, but in some examples the attachment member 160 removably couples the device 100 to a rigid or semi-rigid endoscope) or other such instrument or tool with a working channel and typically some imaging capability as endoscopes usually have (not shown in FIGS. 1 and 2). In some examples, a distal end 165 of the attachment member 160 is adapted to receive, be disposed over, or otherwise be couplable to a portion of the endoscope. In the illustrated example, the distal end 165 of the attachment member 160 defines a recess 167 configured to be coupled to a portion of the endoscope. The attachment member 160 is shown disposed over a portion of the elongated member 110 that is distal to the steering mechanism 130.

The attachment member 160 is adapted to guide longitudinal movement of the steering mechanism 130 (along the axis L for example). At least a portion of the attachment member 160 is disposable within the lumen 132 of the steering mechanism 130. For example, as illustrated in FIG. 2, a guide portion 168 of the attachment member 160 is disposable within at least some of the lumen 132 of the steering mechanism 130. The steering mechanism 130 is movable with respect to the attachment member 160. For example, the steering mechanism 130 can be slidable and/or rotatable with respect to the guide portion 168 of the attachment member 160.

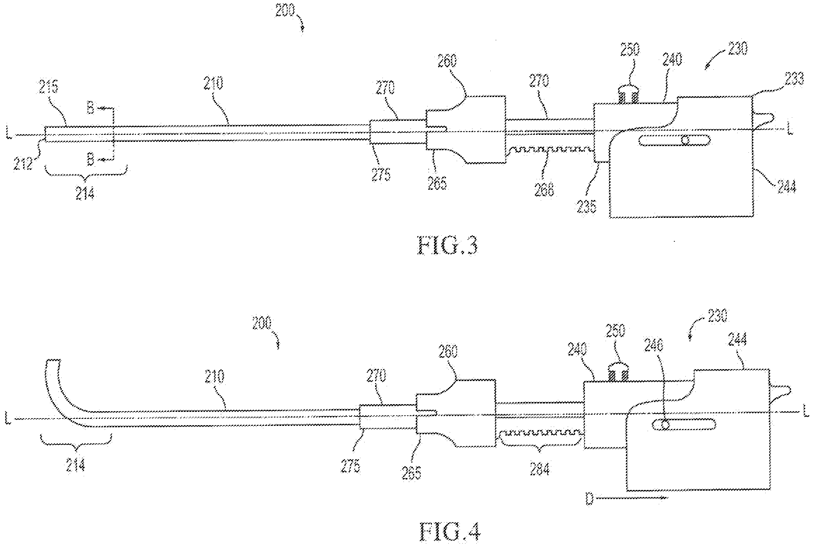

Referring to FIGS. 3-8 and 11-13, another example of a steerable medical device 200 is illustrated. The steerable medical device 200 is adapted to be attached to another medical device or tool, such as a rigid endoscope S, and is adapted to allow for controlled articulation of a portion of the device 200 so that another medical instrument, such as a guidewire G, can be controllably directed to a target location in a body of a patient.

Referring to FIG. 3, the device 200 includes an elongated or tubular member 210, a steering mechanism 230, and an attachment member 260. The tubular member 210 is adapted to be inserted through a working channel of the endoscope. The steering mechanism 230 is adapted to deflect a distal portion of the tubular member 210 towards the target location in the body of the patient so that the advancing distal end of the guidewire (or other instrument) can be controllably directed or guided to the target location. The attachment member 260 is adapted to couple the device 200 to the endoscope.

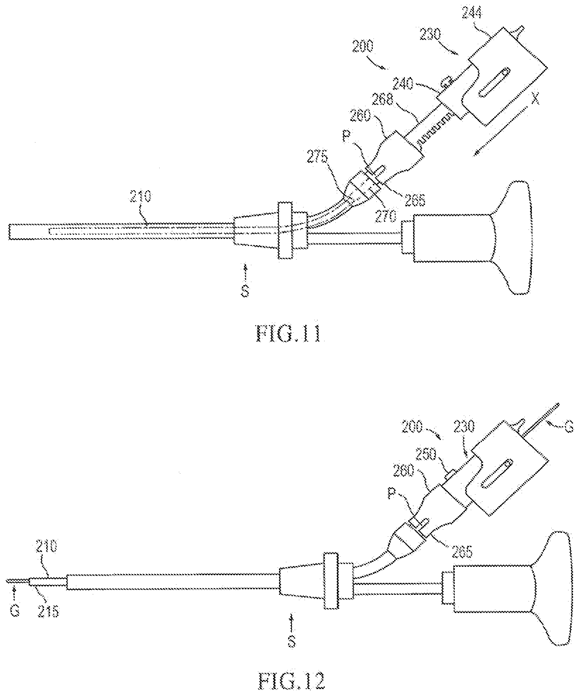

The tubular member 210 can be inserted into the working channel of the endoscope S through a port P of the endoscope, as illustrated in FIG. 11. The tubular member 210 is adapted to receive another medical instrument, such as a guidewire, stone retrieval basket, biopsy tool, laser fiber, or small catheter, for example. The guidewire, for example, can be inserted into the lumen 212 at the proximal end 213 of the tubular member 210. The guidewire can be passed through the lumen 212 of the tubular member 210 until a advancing (or leading) end of the guidewire extends beyond the distal end 215 of the tubular member 210.

The tubular member 210 is also adapted to be controllably articulated such that the tubular member can be used to direct the guidewire (or other instrument) to a target location in the body of the patient. At least a portion of the tubular member 210 is adapted to be deflectable, or steerable. The tubular member 210 includes a proximal end 213 and a distal end 215, and defines a lumen 212 extending between the proximal end and the distal end. The lumen 212 of the elongated member 210 can receive the guidewire (or other instrument).

The elongated member 210 includes a deflectable portion 214 that is adapted to be deflected in at least a first direction. In some examples, the deflectable portion 214 includes the distal end 215 of the elongated member. The deflectable portion 214 of the tubular member 210 allows an operator to target a specific location within the body of the patient. For example, the tubular member 210 of the device 200 can be inserted into a bladder of the patient through the working channel of the endoscope already positioned in the patient's bladder. The operator can then deflect the tubular member such that it approximates the entrance to the patient's ureter, or other place of treatment within the patient's bladder.

The entirety of the tubular member 210 extends along a longitudinal axis L when the deflectable portion 214 is straight or substantially straight, as illustrated in FIG. 3. The deflectable portion 214 of the tubular member 210 can be deflected in a first direction off of (or away from) the longitudinal axis L, as illustrated in FIG. 4.

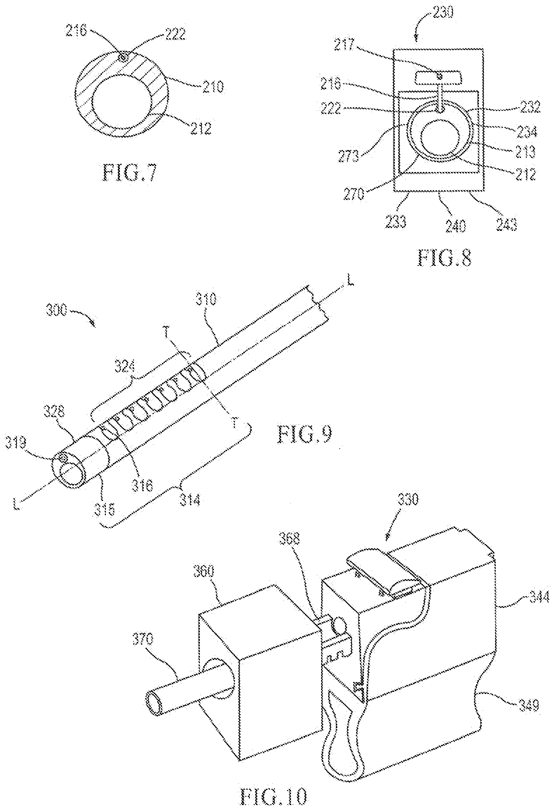

In some examples, the tubular member of a steerable medical device is adapted to reduce deflection resistance in the tubular member. For example, as illustrated in FIG. 9, at least a portion of a tubular member 310, such as a deflectable portion 314, defines at least one of a recess, slot, notch, or opening. The recess, slot, notch, or opening is adapted to help reduce resistance of the tubular member 310 during deflection of the distal end 315 of the tubular member. In the illustrated example, the deflectable portion 314 of the tubular member 310 defines a series of notches 324 (or recesses, slots, or openings). In some examples, each notch of the series of notches 324 extends along an axis different than the longitudinal axis L defined by the tubular member 310. In the example illustrated in FIG. 9, the notches 324 extend along an axis T that is transverse to the longitudinal axis L. In other examples, the deflectable portion of the tubular member is constructed of a material adapted to reduce resistance to deflection, such as a material that is thinner or more flexible that the material of which the remaining portion of the tubular member is constructed.

In some examples not falling within the scope of the claims, as illustrated in FIGS. 7 and 8, the device 200 includes a pull-wire 216. The pull-wire 216 is adapted to be moved by the steering mechanism 230 to move the deflectable portion 214 of the tubular member 210 off of the longitudinal axis L.

In some examples, the lumen 212 defined by the tubular member 210 is a first (or working) lumen and the tubular member 210 further defines a second lumen 222, as illustrated in FIGS. 7 and 8. The second lumen 222 extends from the proximal end 213 of the tubular member 210 to the distal end 215 of the tubular member. The first and second lumens 212, 222 can have varying cross-sectional shapes and/or diameters. For example, the working lumen 212 can be larger than the second 222 lumen. In another example, the working lumen can have a circular cross-sectional shape and the second lumen can have a different cross-sectional shape, such as hexagonal, oval, or square.

The pull-wire 216 can be disposed within the second lumen 222. The pull-wire 216 defines a proximal end 217 and a distal end (not shown in FIGS. 3-8). The proximal end 217 of the pull-wire 216 is coupled to the steering mechanism 230, as illustrated in FIG. 8. The distal end of the pull-wire 216 is coupled to the distal end 215 of the tubular member 210. In some examples, as illustrated in FIG. 9, an attachment ring 328 is disposed on the distal end 315 of the tubular member 310. The distal end 319 of the pull-wire 316 is coupled to the attachment ring 328.

The tubular member can be constructed of any suitable material. For example, the tubular member can be constructed of a biocompatible polymeric material or a thermoplastic elastomer. In another example, the tubular member defining the first and second lumens can be constructed from a Pebax® extrusion.

The tubular member can be constructed of a flexible, semi-rigid, or rigid material. If the tubular member is constructed of a more rigid material, such as Teflon® or nylon, it is beneficial for the deflectable portion of the tubular member to be adapted to decrease deflection resistance, such as by having a series of notches as described above.

Referring to FIGS. 3-8, the steering mechanism 230 of the device 200 is adapted to control movement of the deflectable portion 214 of the tubular member 210. The steering mechanism 230 is adapted to be controlled by a single hand of an operator. For example, a physician can control movement of the steering mechanism 230 with one hand while using the other hand to control a guidewire being inserted into the body of the patient through the tubular member 210.

The steering mechanism 230 includes a proximal end 233 and a distal end 235. In some examples, the steering mechanism 230 is disposed at or over the proximal end 213 of the tubular member 210. At least a portion of the steering mechanism 230 is fixedly coupled to at least a portion of the tubular member 210. For example, the proximal end 233 of the steering mechanism 230 can be fixedly coupled to the proximal end 213 of the tubular member 210. The steering mechanism 230 and tubular member 210 are fixedly coupled such that rotation of the steering mechanism in one direction about the longitudinal axis L correspondingly rotates the elongated member in that one direction about the longitudinal axis. Similarly, movement of the steering mechanism in one longitudinal direction (such as in a proximal or distal direction along the longitudinal axis L) correspondingly moves the elongated member in that one longitudinal direction.

In some examples, at least a portion of the steering mechanism 230 defines an opening or lumen 232, as illustrated in FIG. 8. The lumen 232 of the steering mechanism 230 is adapted to receive at least a portion of the tubular member 210. In the illustrated example, the lumen 232 of the steering mechanism 230 receives (or is disposed over) the proximal end 213 of the tubular member 210.

In some examples, the steering mechanism 230 includes an actuator 244 and a housing 240 (also referred to herein as "housing portion"). In the illustrated example, the actuator 244 is disposed over a portion of the housing 240 of the steering mechanism 230. The actuator 244 is movable with respect to the housing 240, as described in more detail herein.

The actuator 244 is adapted to control movement of the deflectable portion 214 of the tubular member 210 off of the longitudinal axis L. For example, the actuator 244 can be used to direct or control deflection of the deflectable portion 214 of the tubular member 210.

As illustrated in FIGS. 3 and 4, the actuator 244 is movable, with respect to the housing 240, between a first position (FIG. 3) and a second position (FIG. 4). When the actuator 244 is in its first position, the tubular member 210 extends along the longitudinal axis L (or is straight). The actuator 244 is adapted to move the deflectable portion 214 of the tubular member 210 away from the longitudinal axis L as the actuator is moved from its first position towards its second position. In some examples, the actuator 244 is moved to its second position by sliding the actuator in the direction of arrow D, as illustrated in FIG. 4. When the actuator 244 is in its second position, the deflectable portion 214 of the tubular member 210 is off of the longitudinal axis L.

In some examples, the steering mechanism is adapted to limit movement of the actuator. For example, in the illustrated example, a protrusion 246 on the housing 240 is adapted to limit the sliding movement of the actuator 244.

As illustrated in FIG. 10, in some examples, an actuator 344 of a steering mechanism 330 includes a portion 349 adapted to be more easily gripped, grasped, or pulled by an operator. For example, the actuator 344 can include a contoured portion 349 adapted to be gripped by an operator. In other examples, the portion can have a different configuration adapted to allow the user to more easily control actuation of the actuator.

Although the actuator 244 is illustrated as being a slidable actuator disposed over a portion of the housing 240 of the steering mechanism 230, in other examples, the actuator has a different configuration. For example, the actuator can be a slide, button, lever, or another type of actuator disposed on the steering mechanism.

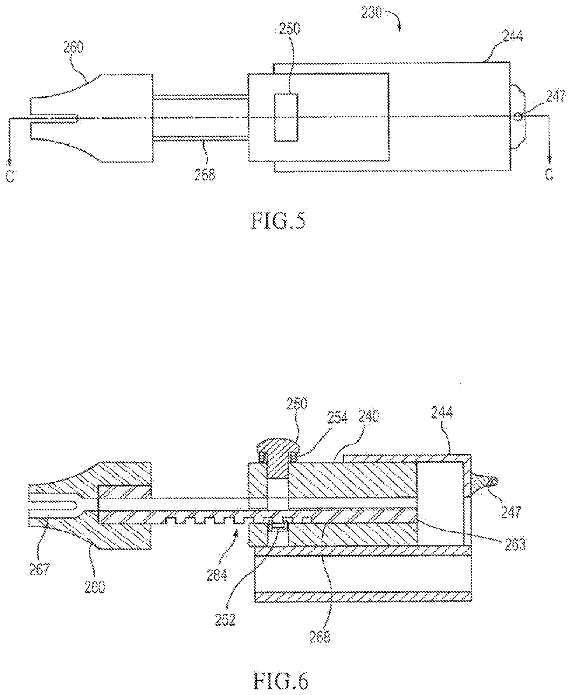

In some examples which do not fall within the scope of the appended claims, at least a portion of the pull-wire 216 is coupled to the actuator 244. For example, as illustrated in FIG. 8, the proximal end 217 of the pull-wire 216 is coupled to the actuator 244 of the steering mechanism 230. In the illustrated example, the pull- wire 216 extends through an opening 247 (illustrated in FIGS. 5 and 6) defined by a portion of the actuator 244. As the actuator 244 is moved towards its second position, the actuator moves (or pulls on) the pull-wire 216 causing the pull-wire to deflect the deflectable portion 214 of the tubular member 210.

Although the device 200 is illustrated and described as including a single pull-wire 216 and as including a tubular member 210 movable in one direction off of the longitudinal axis L, in other examples not falling within the scope of the appended claims, the device can include more than one pull-wire and the tubular member can be movable in more than one direction off of the longitudinal axis L. In one example, the device includes a tubular member that includes a deflectable portion that is moveable in one direction, such as to the right from the perspective of the operator, and another direction different than the one direction, such as to the left from the perspective of the operator. In another example, the deflectable portion of the tubular member is moveable (or deflectable) 360 degrees about the longitudinal axis L. In some examples not falling within the scope of the appended claims, the device includes two, three, four, or more pull-wires adapted to move the tubular member off of the longitudinal axis L. In some examples, the tubular member defines more than two lumens. For example, the tubular member can define four lumens, such as to accommodate four pull-wires, which, however, does not fall within the scope of the claims.

The housing 240 of the steering mechanism 230 includes a proximal end 243 and a distal end 245. In some examples, the housing 240 defines the opening or lumen 232 of the steering mechanism 230. In some examples, the lumen 232 extends from a proximal opening 234 at the proximal end 243 of the housing 240 to a distal opening 236 at the distal end 245 of the housing.

The proximal end 213 of the tubular member 210 is disposed in (or received in) the lumen 232 of the housing 240. The lumen 212 of the tubular member 210 is accessible through the proximal opening 243 of the housing 240. For example, a guidewire, stone retrieval basket, biopsy tool, laser fiber, small catheter, or another medical instrument can be inserted into the lumen 212 of the tubular member 210 through the proximal opening 243 of the housing 240.

In some examples, the housing 240 is the portion of the steering mechanism 230 fixedly coupled to the tubular member 210. For example, the proximal end 243 of the housing 240 can be fixedly coupled to the proximal end 213 of the tubular member 210. Because the housing 240 and tubular member 210 are fixedly coupled, when the housing of the steering mechanism 230 is rotated in one direction about the longitudinal axis L, the tubular member correspondingly moves or rotates in that one direction about the longitudinal axis L. Similarly, when the housing 240 of the steering mechanism 230 is moved in one longitudinal direction, for example in a distal direction along the longitudinal axis L, the tubular member correspondingly moves in that one longitudinal direction.

In some examples, the steering mechanism 230 of the device 200 further includes a fastener 250 (also referred to herein as a "position fastener"). The fastener 250 is adapted to fix the position of the steering mechanism 230, and thus the tubular member 210, with respect to the attachment member 260. The fastener 250 has an unlocked position and a locked position. When the fastener 250 is in the unlocked position, the steering mechanism 230 and tubular member 210 are independently movable of the attachment member 260. When the fastener 250 is in its locked position, as illustrated in FIG. 6, the steering mechanism 230 and tubular member 210 are fixed with respect to (or are not independently movable of) the attachment member 260.

The fastener 250 is biased towards its locked position, such as via springs 254. When the fastener 250 is locked, a portion 252 of the fastener engages a portion of the attachment member 260. In the example illustrated in FIG. 6, a portion 252 of the fastener 250 is engaged with or overlays one of a series of teeth 284. To move the tubular member 210 with respect to the attachment member 260, the fastener 250 is pushed downwards towards the housing 240 and the portion 252 of the fastener disengages the tooth.

The fastener 250 allows an operator to selectively longitudinally position the tubular member 210, such as to achieve a certain depth in the body of the patient or extension of the tubular member 210 beyond a distal end of the endoscope or to accommodate variations in lengths of various endoscopes or distal optics equipment, and then fasten or fix the tubular member with respect to the attachment member 260 to prevent further longitudinal movement.

The attachment member 260 of the steerable medical device 200 is adapted to removably couple the device to the endoscope. For example, the attachment member 260 is adapted to removably couple the device 200 to the port of the endoscope. By being removable, the steerable medical device 200 can be coupled to (or attached to) the endoscope and then be removed from the endoscope at the operator's discretion.

When the attachment member 260 is coupled to the endoscope, the attachment member remains substantially stationary with respect to the endoscope when the steering mechanism 230 and the tubular member 210 are moved in at least one of a rotational direction about the longitudinal axis L or a longitudinal direction along the longitudinal axis.

In some examples, the distal end 265 of the attachment member 260 is adapted removably couple to the endoscope. For example, as illustrated in FIG. 6, the distal end 265 of the attachment member 260 defines a recessed portion 267 adapted to be coupled to or disposed over a portion of the endoscope. In some examples, the distal end 265 of the attachment member 260 is adapted to snap onto the port of the endoscope. In other examples, the attachment member 260 is coupled to the endoscope using another known coupling means, including an adhesive, an interference fit, or interlocking recesses, among others.

Once the attachment member 260 of the device 200 is coupled to the endoscope, the operator need not continue to manually support the device because the coupling of the attachment member to the endoscope will support the device. Thus, the operator is able to use one hand to control the actuator 244 of the steering mechanism 230 and the other hand to manipulate the guidewire, or other medical instrument, being inserted into the working channel of the endoscope and into the body of the patient.

The steering mechanism 230 and the tubular member 210 are movably coupled to the attachment member 260. As illustrated in FIGS. 3 and 4, the attachment member 260 can be disposed over and movable with respect to at least a portion of the tubular member 210 distal to the portion of the tubular member over which the steering mechanism 230 is disposed. Thus, when the attachment member 260 is coupled to the endoscope, the steering mechanism 230 and tubular member 210 can be moved with respect to the attachment member. For example, the steering mechanism 230 and tubular member 210 can be slidably movable with respect to the attachment member 260 in a longitudinal direction. In another example, the steering mechanism 230 and tubular member 210 can be rotatably movable with respect to the attachment member 260. The attachment member is adapted to remain substantially stationary with respect to the other medical device when the attachment member is coupled to the endoscope and the steering mechanism and tubular member are moved longitudinally in a direction along the longitudinal axis and/or rotationally about the longitudinal axis. Because the steering mechanism 230 and tubular member 210 are movable with respect to the attachment member 260, the steering mechanism and tubular member can be moved in any longitudinal or rotational direction when the attachment member is coupled to the endoscope, thus allowing for controllable placement of the distal end 215 of the tubular member within the body of a patient.

The attachment member 260 is configured to guide longitudinal movement of the steering mechanism 230 and tubular member 210, for example in at least one of a proximal or a distal direction along the longitudinal axis L. In some examples, at least a portion of the attachment member 260 is received within the steering mechanism 230, such as within an opening or lumen 232 of the steering mechanism. For example, a guide portion 268 of the attachment member 260, which includes the proximal end portion 263 (illustrated in FIG. 6) of the attachment member 260, can be disposed within the lumen 232 of the steering mechanism 230. The steering mechanism 230 is movable over the guide portion 268 of the attachment member 260 received or disposed in the steering mechanism. In some examples, the guide portion 268 (or axial guide) of the attachment member 260 defines a lumen or recess adapted to receive at least a portion of the tubular member 210. For example, as illustrated in FIG. 10, the guide portion 268 can have a semi-circular cross-section, and thus define a recess (the "U" of the semi-circle) adapted to receive a portion of the tubular member. The tubular member 210 is also movable with respect to the guide portion 268 of the attachment member 260.

In some examples, the steerable medical device 200 includes an indicia of the longitudinal position of the distal end 215 of the tubular member 210. For example, the indicia can indicate a depth of insertion of the tubular member 210 into the body of the patient by corresponding to a length of extension of the distal end 215 of the tubular member 210 beyond a distal end of the endoscope. For example, as illustrated in FIGS. 4 and 6, the device 200 includes an indicia that is a series of protrusions or teeth 248. Each protrusion (or tooth) corresponds to a measurement of the depth extension of the tubular member 210 beyond the distal end of the endoscope and into the body of the patient.

In the illustrated example, the indicia 284, the series of teeth 284 that engage the fastener 250, and the guide 268 are the same piece of the device 200 having multiple functions. In other examples, however, the indicia is different than the teeth configured to engage the fastener and/or the guide. For example, the indicia can be included on or disposed elsewhere on the device 200. In other examples, the device can include an index or position indexer upon which the indicia is disposed, and the index or position indexer can be coupled to at least one of the steering mechanism, tubular member, or the attachment member. Although the indicia is illustrated as a series of protrusions, in other examples, the indicia can be one or a series of lines, ridges, numbers, colors, or any other visual or tactile indicia corresponding to a depth of insertion of the tubular member.

In some examples, as illustrated in FIGS. 3 and 4, the steerable medical device 200 includes a reinforcement (or stiffener) shaft 270. The reinforcement shaft 270 is adapted to reinforce at least a portion of the tubular member 210. For example, the reinforcement shaft 270 provides reinforcement or support to the portion of the tubular member 210 that is inserted into the port of the endoscope. The reinforcement shaft 270 includes a proximal end 273 and a distal end 275 and defines a lumen (not shown) extending from the proximal end to the distal end of the reinforcement shaft.

The reinforcement shaft 270 is disposable over at least a portion of the tubular member 210. For example, the lumen of the reinforcement shaft 270 is adapted to receive a portion of the tubular member 210. In some examples, as illustrated in FIG. 8, a portion, such as the proximal end 273, of the reinforcement shaft 270 is disposed within the lumen 232 of the steering mechanism 230. In some examples, the proximal end 273 of the reinforcement shaft 270 is coupled to the proximal end 233 of the steering mechanism 230 and to the proximal end 213 of the tubular member 210. In some examples, the reinforcement shaft 270, tubular member 210, and steering mechanism 230 are fixedly coupled together such that when one is rotated or moved longitudinally about or along the longitudinal axis L, each of the others is correspondingly rotated or moved longitudinally about or along the longitudinal axis L. In other examples, as illustrated in FIG. 10, a reinforcement shaft 370 does not extend into the steering mechanism 330, but only reinforces the portion of the tubular member (not shown) extending through the attachment member 360 and entering into the port of the endoscope.

A portion of the reinforcement shaft 270 is adapted to be inserted into the endoscope. In some examples, the distal end 275 of the reinforcement shaft 270 is adapted to be inserted into, or extend telescopically into, the endoscope, such as into the port P of the endoscope S, as illustrated in dashed lines in FIG. 11.

A steerable medical device according to the invention can be used to perform or assist in a variety of medical procedures. For example, the steerable device 200 can be used in procedures to treat conditions in the upper urinary tract of a patient, such as kidney stones, or in the bladder of a patient, such as tumors. Referring to FIGS. 11 through 14, a medical device, such as endoscope S, is inserted into the patient's body. For example, in some procedures, the endoscope is inserted into a bladder of the patient. The tubular (or elongated) member 210 of the steerable medical device 200 (shown in dashed lines in FIG. 11) is at least partially inserted into the working channel of the endoscope S through port P.

The attachment member 260 of the device 200 removably couples the device to the endoscope S. As illustrated in FIG. 12, the fastener 250 of the steering mechanism 230 is moved from its locked to its unlocked position and the steering mechanism 230 is moved in a distal direction (indicated by the arrow X in FIG. 11) with respect to the attachment member 260. Movement of the steering mechanism 230 distally when the fastener 250 is unlocked advances the tubular member 210 until its distal end 215 extends beyond a distal end of the endoscope S. The steering mechanism 230, and thus the tubular member 210, can be alternatively moved distally and proximally until the operator achieves a desired extension of the distal end 215 of the tubular member 210 beyond the distal end of the endoscope S.

A guidewire G is inserted into the working lumen 212 of the tubular member 210 via the proximal opening of the steering mechanism 230. The guidewire G is passed through the lumen 212 of the tubular member 210 until a distal end of the guidewire is at or near the distal end 215 of the tubular member.

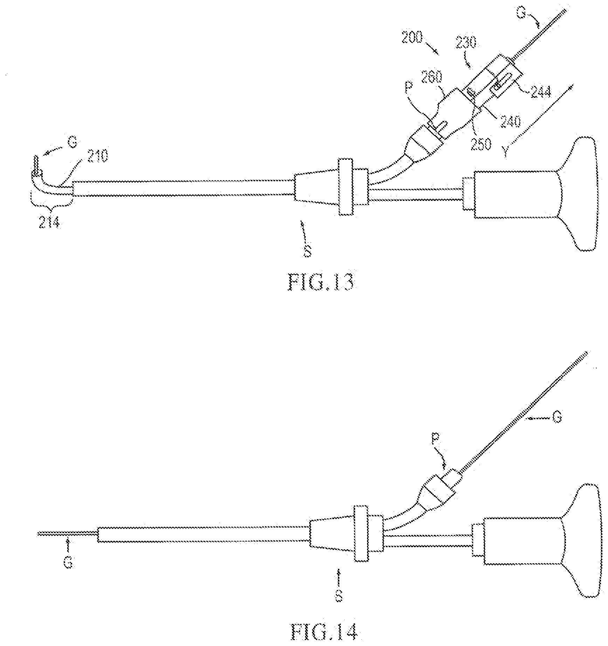

Referring to FIG. 13, showing an example not falling within the scope of the appended claims, the actuator 244 of the steering mechanism 230 is moved in the direction of arrow Y to its second position, and the deflectable portion 214 of the tubular member 210 is moved away from the longitudinal axis. The actuator 244 moves a pull wire (not shown in FIG. 13) to deflect the deflectable portion 214 of the tubular member 210 off of the longitudinal axis. The steering mechanism 230 is partially rotated in one direction with respect to the attachment member 260 (and the longitudinal axis) towards the handle of the scope (i.e., in a counterclockwise direction), and therefore the tubular member 210 is partially rotated in the one direction. The steering mechanism and tubular member can be rotated in clockwise and counterclockwise directions until the deflected distal end of the tubular member faces or approximates the target location of the body of the patient. If necessary, the tubular member can be readjusted in a proximal or distal direction to better approximate the deflected distal end of the tubular member to the target location of the patient's body.

The ability to control deflection, rotation, and longitudinal position of the tubular member allows the physician (or other operator) to introduce the guidewire G, or other medical instrument, to a target location within the body of the patient. For example, the physician can manipulate the tubular member 210 until the guidewire G is positioned at the entrance to the patient's ureter. Furthermore, the physician can control the deflection, rotation, and longitudinal position of the tubular member with one hand, leaving the other hand free to manipulate the guidewire.

With the guidewire G positioned at the target location, the attachment member 260 is decoupled (or removed) from the port P and the steerable medical device 200 is removed in the direction of arrow Y, as indicated in FIG. 13, from the body of the patient and from the endoscope S while leaving the guidewire G substantially in position at the target location in the body of the patient. The device 200 can be removed over the guidewire G or other medical device, leaving the guidewire G or other medical device available in the endoscope S for further treatment procedures, as illustrated in FIG. 14.

Although use of the steerable medical device in a medical procedure has been illustrated and described herein as occurring in one order, in other procedures the steps can occur in a different order. For example, the steering mechanism 230 and tubular member 210 can be longitudinally and/or rotationally positioned before the distal end 215 of the tubular member is deflected.

Additionally, although the steerable medical device has been illustrated and described herein mostly as being used in conjunction with another medical device (such as a rigid endoscope) and through a working channel of that other device, a steerable medical device according to examples of the present disclosure can be used to controllably direct a guidewire or other instrument without passing through the working channel of another device.

In some examples, the steerable medical device 200 is a guiding catheter adapted to be disposable after a single-use. After the operator has used the guiding catheter to position the guidewire, or other medical instrument, in the body of the patient, the operator can remove the guiding catheter from the body of the patient and discard it.

As described above, a steerable medical device as described herein can be configured to receive an optical fiber for use in the delivery of laser energy to a target location within a patient. Various example embodiments of a laser-energy delivery device are described below.

A laser-energy-delivery device can be configured to receive laser energy emitted (also can be referred to as being launched) from a laser energy source. Specifically, the laser-energy delivery device can receive the laser energy at a connector portion of the laser-energy-delivery device. The connector portion can be at a proximal end portion (can be referred to as an entry end portion) of the laser-energy-delivery device. In some embodiments, the connector portion can be referred to as a launch connector portion or as a launch connector because laser energy can be emitted into (e.g., launched into) the connector portion. The laser-energy-delivery device can also include an optical fiber coupled to the connector portion of the laser-energy delivery device. Laser energy can be propagated within the optical fiber coupled to the connector portion until the laser energy is transmitted from the distal end of the optical fiber toward, for example, a target treatment area within a body of a patient. The connector portion can include a doped silica component that has an inner surface heat-fused to an outer portion of the optical fiber. All or substantially all of the surface area of the inner surface of the doped silica component can be heat-fused to the outer portion of the optical fiber. In some embodiments, the doped silica component can be referred to as a doped silica capillary or as a doped silica ferrule.

The optical fiber can be a silica-based optical fiber and can include, for example, a fiber core, one or more cladding layers (e.g., a cladding layer disposed around the fiber core), a buffer layer (e.g., a buffer layer disposed around a cladding layer), and/or a jacket (e.g., a jacket disposed around a buffer layer). In some embodiments, a numerical aperture of the fiber core with respect to one or more cladding layers around the fiber core can be between 0.1 and 0.3. In some embodiments, a numerical aperture of the cladding layer(s) with respect to the buffer layer can be between 0.2 and 0.6. At least a portion of the cladding layer(s), the buffer layer, and/or the jacket can be stripped from the optical fiber before the doped silica component is heat-fused to the optical fiber. At least a portion of the doped silica component (e.g., the inner surface of the doped silica component) can have an index of refraction lower than an index of refraction associated with the outer portion of the optical fiber. The doped silica component can be doped with a concentration of a dopant (e.g., a fluorine dopant, a chlorine dopant, a rare-earth dopant, an alkali metal dopant, an alkali metal oxide dopant, etc.) that can, at least in part, define the index of refraction of the doped silica component.

Because of the difference in the respective indices of refraction of the doped silica component and the outer portion of the optical fiber (e.g., cladding layer), laser energy (e.g., stray laser energy) from within the optical fiber and incident on an interface defined by the doped silica component and the outer portion of optical fiber is totally or substantially totally internally reflected within the optical fiber. In some embodiments, stray laser energy that is, for example, not totally or substantially totally internally reflected can be absorbed within the doped silica component.

A proximal end of the connector end portion of the laser-energy delivery device can be defined so that it is flat and within a plane that is substantially normal to a longitudinal axis (or centerline) of the laser-energy delivery device. In some embodiments, the doped silica component can be formed from, for example, a doped silica pre-form before being fused to an optical fiber. The connector portion of the laser-energy delivery device can be coupled to (e.g., adhesively bonded to, press fit with) a component such as a metal ferrule, a housing, and/or a grip member. In some embodiments, the optical fiber can have a spherical distal end portion, a straight-firing distal end portion, or can have a side-firing distal end portion.

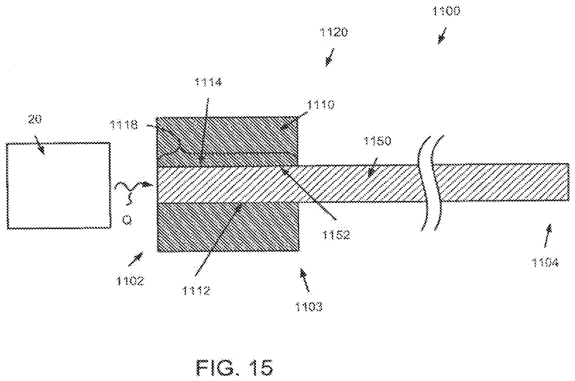

FIG. 15 is a schematic diagram of a side cross-sectional view of a connector portion 120 of a laser-energy-delivery device 1100, according to an example. The laser-energy delivery device 1100 can be associated with (e.g., used in conjunction with) an endoscope (not shown). The connector portion 1120 of the laser-energy delivery device 1100, which is at a proximal end portion 1102 of the laser-energy delivery device 1100 (also a proximal end portion 1102 of a doped silica component 1110), is configured to receive laser energy Q emitted from a laser energy source 20. The laser energy source 20 can be, for example, a holumium (Ho) laser source, a holumium:YAG (Ho:YAG) laser source, a neodymium-doped:YAG (Nd:YAG) laser source, a semiconductor laser diode, and/or a potassium-titanyl phosphate crystal (KTP) laser source. In some embodiments, the numerical aperture of laser energy emitted from the laser energy source 20 can be between 0.1 and 0.4. The laser energy Q can be associated with a range of electromagnetic radiation from an electromagnetic radiation spectrum.

The laser energy Q emitted from the laser energy source 20 and received at the connector portion 1120 of the laser-energy delivery device 1100 can be propagated along an optical fiber 1150 until at least a portion of the laser energy Q is transmitted from a distal end portion 1104 of the laser-energy delivery device 1100. In other words, the optical fiber 1150 can function as a wave-guide for the laser energy Q.

The optical fiber 1150 can be a silica-based optical fiber and can have, for example, a fiber core (not shown in FIG. 15). In some embodiments, the fiber core can be made of a suitable material for the transmission of laser energy Q from the laser energy source 20. In some embodiments, for example, the fiber core can be made of silica with a low hydroxyl (OH⁻) ion residual concentration. Laser energy wavelengths ranging from about 500 nm to about 2100 nm can be propagated within the fiber core during a surgical procedure. An example of low hydroxyl (low-OH) fibers used in medical devices is described in U.S. Patent No. 7,169,140 to Kume. The fiber core can be a multi-mode fiber core and can have a step or graded index profile. The fiber core can also be doped with a concentration of a dopant (e.g., an amplifying dopant).

The optical fiber 1150 can also have one or more cladding layers (not shown in FIG. 15) and/or a buffer layer (not shown in FIG. 15) such as an acrylate layer. The fiber core and/or cladding layer(s) can be pure silica and/or doped with, for example, fluorine. The cladding can be, for example, a single or a double cladding that can be made of a hard polymer or silica. The buffer layer can be made of a hard polymer such as Tefzel®, for example. When the optical fiber 1150 includes a jacket (not shown in FIG. 15), the jacket can be made of Tefzel®, for example, or can be made of other polymer-based substances.

Although not shown in FIG. 15, the laser energy source 20 can have a control module (not shown) configured to control (e.g., set, modify) a timing, a wavelength, and/or a power of the emitted laser energy Q. In some embodiments, the laser energy Q can have a power of between 1 watt and 10 kilowatts. In some embodiments, the control module can also be configured to perform various functions such as laser selection, filtering, temperature compensation, and/or Q-switching. The control module can be a hardware-based control module and/or a software-based control module that can include, for example, a processor and/or a memory.

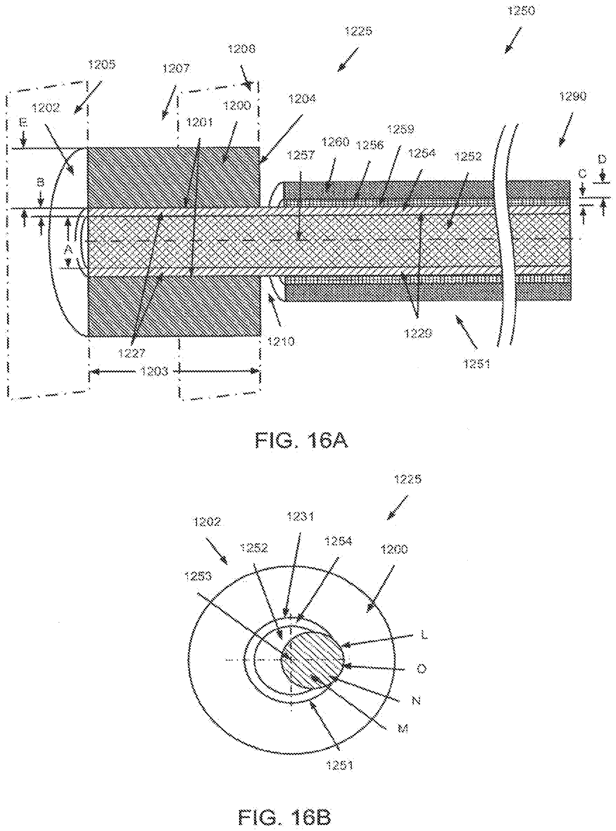

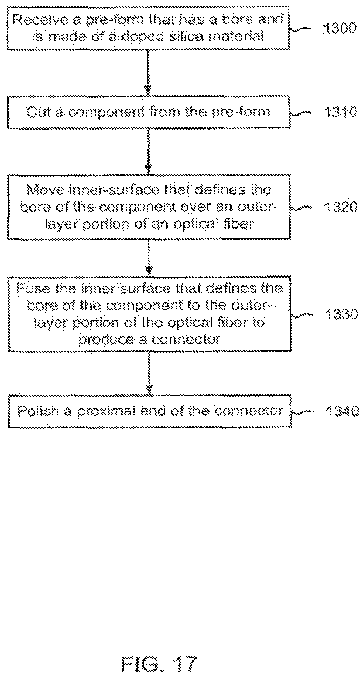

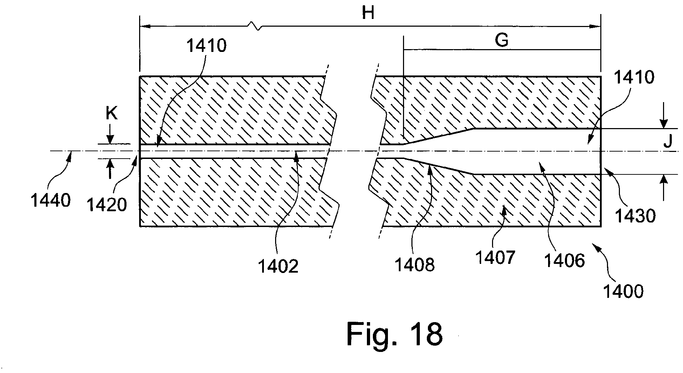

The connector portion 1120 has a doped silica component 1110 fused to the optical fiber 1150 at the proximal end portion 1102 of the laser-energy delivery device 1100. As shown in FIG. 15, the optical fiber 1150 is disposed within at least a portion of the doped silica component 1110. In some embodiments, the doped silica component 1110 can be referred to as a doped silica ferrule, a doped silica capillary, or a doped silica tube. More details related to the dimensions of the doped silica component 1110 and the optical fiber 1150 are described in connection with FIG. 16 and FIG. 17. In some embodiments, a metal ferrule (not shown in FIG. 15) or a housing (not shown in FIG. 2), for example, can be coupled to the doped silica component 1110. More details related to components that can be coupled to the doped silica component 1110 are described in connection with FIGS. 18 through 22B.

The doped silica component 1110 is doped such that an index of refraction of at least an inner surface 1114 of the doped silica component 1110 is lower than or equal to an index of refraction of an outer surface 1152 of the optical fiber 1150. In some embodiments, the doped silica component 1110 can be doped with a concentration of fluorine. In some embodiments, the doped silica component 1110 can be uniformly doped or doped in a non-uniform (e.g., graded) fashion. Because of the difference in the indices of refraction, a portion of the laser energy Q propagated within the optical fiber 1150 and incident on an interface 1112 defined by the inner surface 1114 of the doped silica component 1110 and the outer surface 1152 of the optical fiber 1150 can be totally or substantially totally internally reflected within the optical fiber 1150. If the optical fiber 1150 has a cladding layer (not shown), a portion of the laser energy Q propagated within the cladding layer and incident on the interface 1112 can be totally or substantially totally internally reflected within the cladding layer. If the index of refraction of the doped silica component 1110 were, for example, substantially equal to that of the outer surface 1152 of the optical fiber 1150, an undesirable (e.g., a damaging) percentage of the laser energy Q could be transmitted into the doped silica component 110 and into, for example, surrounding components.

In some embodiments, the interface 1112 can be configured to redirect a portion of the laser energy Q (e.g., stray laser energy) emitted near the interface 1112 because of, for example, misalignment of the laser energy source 20 with the connector portion 1120. In some embodiments, a portion of the laser energy Q emitted directly into the doped silica component 1110 can be at least partially absorbed within the doped silica component 1110. Misalignment can be caused by improper alignment of the laser energy source 20 with the connector portion 1120. Misalignment can also be caused by drift in targeting of emitted laser energy Q by the laser energy source 20 and/or thermo-lensing effects associated with the laser energy source 20.

During manufacture, at least a portion of the doped silica component 1110 is heat-fused to the optical fiber 1150. Specifically, at least a portion of the doped silica component 1110 and the optical fiber 1150 are heated so that the inner surface 1114 of the doped silica component 1110 is fused to the outer surface 1152 of the optical fiber 1150. In some embodiments, multiple areas (e.g., longitudinally discontinuous) along a length 1118 of the doped silica component 1110 can be heat-fused to the optical fiber 1150. The areas may or may not continuously surround (e.g., circumferentially surround) the optical fiber 1150. For example, a portion of the doped silica component 1110 near or at the proximal end portion 1102 of the doped silica component 1110 and/or a portion of the doped silica component 1110 near or at a distal end 1103 of the doped silica component 110 can be heat-fused to the optical fiber 1150. In some embodiments, a top surface area portion and/or a bottom surface area portion of the optical fiber 1150 can be heat-fused to the inner surface 1114 of the doped silica component 1110 without heat-fusing the remaining portions (e.g., the bottom surface area portion of the top surface area portion, respectively). More details related to a method for heat-fusing the doped silica component 1110 to the optical fiber 1150 are described in connection with FIG. 17.

In some embodiments, the doped silica component 1110 can be made separately from the optical fiber 1150 and shaped so that the optical fiber 1150 can be inserted into the doped silica component 1110. For example, in some embodiments, the doped silica component 1110 can have a cylindrical shape and a circular bore (e.g., a lumen) within which the optical fiber 1150 can be inserted.

In some embodiments, the laser-energy delivery device 1100 can be used within an endoscope (not shown) that can define one or more lumens (sometimes referred to as working channels). In some embodiments, the endoscope can include a single lumen that can receive therethrough various components such as the laser-energy delivery device 1100. The endoscope can have a proximal end configured to receive the distal end portion 1104 of the laser-energy delivery device 1100 and a distal end configured to be inserted into a patient's body for positioning the distal end portion 1104 of the laser-energy delivery device 1100 in an appropriate location for a laser-based surgical procedure. The endoscope can include an elongate portion that can be sufficiently flexible to allow the elongate portion to be maneuvered within the body. In some embodiments, the endoscope can be configured for use in a ureteroscopy procedure.

The endoscope can also be configured to receive various medical devices or tools through one or more lumens of the endoscope, such as, for example, irrigation and/or suction devices, forceps, drills, snares, needles, etc. An example of such an endoscope with multiple lumens is described in U.S. Patent No. 6,296,608 to Daniels et al. In some embodiments, a fluid channel (not shown) is defined by the endoscope and coupled at a proximal end to a fluid source (not shown). The fluid channel can be used to irrigate an interior of the patient's body during a laser-based surgical procedure. In some embodiments, an eyepiece (not shown) can be coupled to a proximal end portion of the endoscope, for example, and coupled to a proximal end portion of an optical fiber that can be disposed within a lumen of the endoscope. Such an embodiment allows a medical practitioner to view the interior of a patient's body through the eyepiece.

FIG. 16A is a schematic diagram of a side cross-sectional view of a connector portion 1225 of a laser-energy delivery device 1250, according to an example. The laser-energy delivery device 1250 includes an optical fiber 251. As shown in FIG. 16A, a doped silica capillary 1200 is heat-fused to a first portion 1227 of a cladding layer 1254 of the optical fiber 1251. The first portion 1227 is at a proximal end portion 1207 of the optical fiber 1251. The cladding layer 1254 is disposed around a fiber core 1252 of the optical fiber 1251. A coating 1256 is disposed around a second portion 1229 of the cladding layer 1254 of the optical fiber 1251 and a jacket 1260 is disposed around the coating 1256. In some examples, the coating 1256 can be, for example, an acrylate coating such as a fluorinated acrylate coating. The coating 1256 can also be referred to as a buffer layer. In some examples, the jacket 1260 can be made of a polymer-based material such as an ethylene tetrafluoroethylene (ETFE) copolymer and/or a nylon-based material. The second portion 1229 of the cladding layer 1254 is distal to the first portion 1227 of the cladding layer 1254. In some examples, the optical fiber 1251 can have multiple cladding layers (not shown).

Laser energy (not shown) emitted into the connector portion 1225 of the laser-energy delivery device 1250 can be propagated along the optical fiber 1251 and transmitted out of a distal end 1290 of the optical fiber 1251. Although the portions (e.g., cladding layer 1254) included within the laser-energy delivery device 1250 can have a variety of cross-sectional shapes such as ovals, and so forth, the portions are shown and described as circular-shaped portions.

In some examples, the doped silica capillary 1200 can have a length 1203 of, for example, 1 centimeter (cm) to 8 cm. In some examples, the length 1203 of the doped silica capillary 1200 can be less than 1 cm. In some examples, the length 1203 of the doped silica capillary 1200 can be greater than 8 cm. In this example, the entire length 1203 of an inner surface 1201 of the doped silica capillary 1200 is heat-fused to the cladding layer 1254 of the optical fiber 1251. In some examples, the heat-fused portion (e.g., the heat-fused area) can be less than the entire length 1203 of the doped silica capillary 1200. In some examples, the length of the heat-fused portion can vary depending on the length 1203 of the doped silica capillary 1200. For example, if the doped silica capillary 1200 is greater than 3 cm, less than the entire length 1203 of the doped silica capillary 1200 can be heat-fused to the cladding layer 1254.

The fiber core 1252 of the optical fiber 1251 can have an outer diameter A, for example, between approximately 20 micrometers (µm) to 1200 µm. The cladding layer 1254 of the optical fiber 1251 can have a thickness B, for example, between approximately 5 µm to 120 µm. In some examples, the outer diameter (not shown) of the cladding layer 1254 can be 1 to 1.3 times the outer diameter A of the fiber core 1252 of the optical fiber 1251.

The coating 1256 of the optical fiber 1251 can have a thickness C, for example, between approximately 5 µm to 60 µm. The thickness of the coating 1256 of the optical fiber 1251 can be defined to increase the mechanical strength of the optical fiber 1251 during flexing of the optical fiber 1251. The jacket 1260 of the optical fiber 1251 can have a thickness D, for example, between approximately 5 µm to 500 µm. The doped silica capillary 1200 can have a thickness E, for example, between 20 µm and several millimeters (mm).

The doped silica capillary 1200 can be cut from a doped silica pre-form and heat-fused to the first portion 1227 of the cladding layer 1254 after portions of the coating 1256 and the jacket 1260 are stripped from the first portion 1227 of the cladding layer 1254. A relatively strong bond that is resistant to tensile forces (e.g., forces in the direction of a longitudinal axis 1257 (or centerline) of the optical fiber 1251) can be formed between the doped silica capillary 1200 and the cladding layer 1254 when they are heat-fused together. The doped silica capillary 1200 and the cladding layer 1254 can be heat-fused so that structural failure (e.g., separation) caused, for example, by shearing strain at specified tensile force levels can be substantially avoided. In other words, the heat-fused area can be sufficiently large to provide mechanical stability (e.g., resistance to shear forces) between the cladding layer 1254 and the doped silica capillary 1200. For example, the cladding layer 1254 with a diameter of approximately 150 µm can be heat-fused with the doped silica capillary 1200 so that the cladding layer 1254 will not separate from the doped silica capillary 1200 when up to approximately 13.3 N (3 pounds of force; e.g., tensile force) is applied between the doped silica capillary 1200 and the cladding layer 1254.

In this example, an index of refraction of the doped silica capillary 1200 is lower than an index of refraction of the cladding layer 1254. Also, the index of refraction of the cladding layer 1254 is lower than an index of refraction of the fiber core 1252. The coating 1256 has an index of refraction that is lower than the index of refraction of the cladding layer 1254. In some examples, the coating 1256 can have an index of refraction that is higher, lower, or substantially the same as the index of refraction of the doped silica capillary 1200.

As shown in FIG. 16A, a proximal end 1202 of the connector portion 1225 of the laser-energy delivery device 1250 is within a single plane 1205. The plane 1205 is substantially normal to the longitudinal axis 1257 (or centerline) of the optical fiber 1251. In other words, the proximal end 1202 of the connector portion 1225 of the laser-energy delivery device 1250 is flat or substantially flat. After the doped silica capillary 1200 is heat-fused to the cladding layer 1254, the proximal end 1202 of the connector portion 1225 of the laser-energy delivery device 1250 can be modified (e.g., mechanically polished, modified using laser energy) until it is flat or substantially flat.

Although not shown, in some examples, the proximal end 1202 of the connector portion 1225 of the laser-energy delivery device 1250 can have a lens. For example, a lens can be coupled (e.g., bonded, fused) to the proximal end 1202. In some examples, a lens can be formed from the doped silica capillary 1200, cladding layer 1254, and/or, fiber core 1252 of the optical fiber 1251.

Although not shown, in some examples, the proximal end 1202 of the connector portion 1225 is not flat. In some examples, the cladding layer 1254 and/or the fiber core 1252 can be configured to protrude proximal to a proximal end of the doped silica capillary 1200. In other words, a proximal portion of the cladding layer 1254 and/or a proximal portion of the fiber core 1252 can protrude proximal to the proximal end 1202 of the connector portion 1225, which is within plane 1205. In some examples, a proximal end of the doped silica capillary 1200 is configured to protrude proximally over a proximal end of the cladding layer 1254 and/or a proximal end of the fiber core 1252. In other words, the proximal end of the doped silica capillary 1200, the proximal end of the cladding layer 1254, and/or the proximal end of the fiber core 1252 can be within different planes. In some examples, the different planes can be non-parallel.

As shown in FIG. 16A, an air gap 1210 is disposed between the doped silica capillary 1200 and portions of the layers (e.g., the coating 1256) disposed around the cladding layer 1254. Specifically the air gap 1210 is disposed between the doped silica capillary 1200 and the coating 1256 as well as the jacket 1260. In some examples, the coating 1256 and/or the jacket 1260 may be coupled to (e.g., in contact with, bonded to, fused to) the doped silica capillary 1200.

As shown in FIG. 16A, a distal end 1204 of the doped silica capillary 1200 can be substantially flat and within a plane 1208 parallel to plane 1205. Although not shown, in some examples, the distal end 1204 of the doped silica capillary 1200 can have one or more surfaces non-parallel to plane 1208. For example, at least a portion of the distal end 1204 can have a concave portion and/or a convex portion. An example of a doped silica capillary 1200 having a concave portion is described in connection with FIG. 18.

In some examples, the doped silica capillary 1200 can be a monolithically formed component. In some examples, the doped silica capillary 1200 can include multiple separate portions (e.g., discrete or discontinuous sections) that are individually or collectively fused to define the doped silica capillary 1200. For example, the doped silica capillary 1200 can include tubular sections that are serially disposed over the cladding layer 1254. The tubular sections can be fused to one another as well as the cladding layer 1254 of the optical fiber 1251.

In some examples, a numerical aperture of laser energy guided within a portion of the optical fiber 1251 proximal to plane 1208 is substantially equal to a numerical aperture of laser energy guided within a portion of the optical fiber 1251 disposed distal to plane 1208. In some examples, the numerical aperture associated with a proximal end of the optical fiber 1251 can be substantially unchanged along the fiber core 1252 (and/or the cladding layer 1254) disposed within the doped silica component 1200. In some examples, the numerical aperture of the fiber core 1252 along substantially the entire length of the optical fiber 1251 is substantially constant. Thus, the optical fiber 1251 can have a smaller bend diameter with substantially less laser energy leaked into, for example, the cladding layer 254 than if the numerical aperture of the optical fiber 1251 were to increase along, for example, the doped silica component 200 (from the proximal end toward the distal end).

FIG. 16B is a schematic diagram of the proximal end 202 of the connector portion 225 shown in FIG. 6A, according to an example. As shown in FIG. 16B, a cross-sectional area L of laser energy emitted into the connector portion 1225 is offset from a center 1253 of the fiber core 1252 of the optical fiber 1251. The cross-sectional area L of the laser energy can be referred to as a laser spot or as a focal point spot. A portion M of the cross-sectional area L of the laser energy is emitted into the fiber core 1252, a portion N of the cross-sectional area L of the laser energy is emitted into the cladding layer 1254, and a portion O of the cross-sectional area L of the laser energy is emitted into the doped silica capillary 1200. In some examples, the laser spot can have a diameter between 20 microns and 500 microns.

As shown in FIG. 16B, the doped silica capillary 1200 and cladding layer 1254 define an interface 1231. Because the index of refraction of the doped silica capillary 1200 is lower than the index of refraction of the cladding layer 1254, the interface 1231 totally or substantially totally internally reflects laser energy from within the cladding layer 1254 and incident on the interface 1231. Thus, the portion N of the laser energy that is emitted into the cladding layer 1254 and incident on the interface 1231 is totally or substantially totally internally reflected into the cladding layer 1254 rather than transmitted into the doped silica capillary 1200. The index of refraction of the doped silica capillary 1200 and the index of refraction of the cladding layer 1254 can be defined so that the interface 1231 totally or substantially totally internally reflects incident laser energy at a desirable level.

The portion O of the cross-sectional area L of the laser energy that is directly emitted into the doped silica capillary 1200 can be substantially absorbed or totally absorbed within the doped silica capillary 1200 and/or dissipated in the form of heat. The doping concentration of the doped silica capillary 1200 can be defined so that laser energy, such as laser energy, is absorbed and/or dissipated in the form of heat within the doped silica capillary 1200 at a desirable rate.

Referring back to FIG. 16A, in some examples, at least a portion of laser energy can be emitted into the cladding layer 1254 of the connector 1225, for example, due to slight misalignment or spatial drift of the laser related to the laser-energy delivery device 1250. The cladding layer 1254 can be used, along with the fiber core 1252, as a transmission medium of the laser energy at least over the length 1203 of the doped silica capillary 1200. In some examples, laser energy emitted into the cladding layer 1254 of the connector 1225 can be initially guided by the interface 1231 (shown in FIG. 16B) between the cladding layer 1254 and the doped silica capillary 1200. In some examples, the laser energy launched into the cladding layer 1254 of the connector 1225 can be reflected (e.g., guided) into the fiber core 1252 by the interface 1231 between the cladding layer 1254 and the doped silica capillary 1200 over the length 1203 of the doped silica capillary 1200. In other words, laser energy launched into the cladding layer 1254 of the connector can migrate into the fiber core 1252, for example, over the length 1203 of the doped silica capillary 1200. Thus, undesirable effects associated with overfill of laser energy within the cladding layer 1254 during operation can be substantially reduced or avoided. When laser energy is emitted into the cladding layer 1254 as well as the fiber core 1252, the cladding layer 1254 and fiber core 1252 effectively collectively function as a fiber core, and the doped silica capillary 200 effectively functions as a cladding layer. If necessary, residual laser energy that is not reflected into the fiber core 252 by the interface 1231 between the cladding layer 1254 and the doped silica capillary 1200 (within length 1203) can be guided by an interface 1259 between the cladding layer 1254 and the coating 1256.

As shown in FIG. 16A, the fiber core 1252 (and cladding layer 1254) of the connector portion 1225 is substantially straight (not tapered). Even though the fiber core 1252 of the connector portion 1225 is substantially straight, the connector portion 1225 can capture and guide more laser energy in the fiber core 1252 and/or the cladding layer 1254 than a fiber core connector portion with a tapered fiber core (not shown) for a given fiber core size and for a given laser spot size / numerical aperture. One reason this can be achieved is because of the laser energy reflective properties provided by the interface 1231 between the cladding layer 1254 and the doped silica capillary 1200 of the connector portion 1225. The substantially straight fiber core 1252 (and cladding layer 1254) of the connector portion 1225 may not modify the effective numerical aperture of laser energy emitted into the fiber core 1252 (and/or cladding layer 1254) in an undesirable fashion. Thus, laser energy can be substantially guided within the fiber core 1252 (and/or cladding layer 1254) without penetrating the cladding layer 1254 (if the effective numerical aperture of the laser energy were increased by, for example, tapering). In addition, undesirable overfill of the cladding layer 1254 caused by bending of the fiber core 1252 (which reduces the effective cone angle of laser energy relative to the cladding-coating interface 1259) of the laser-energy delivery device 1250 during operation can be substantially reduced or avoided. This can be substantially reduced or avoided because the effective cone angle of laser energy relative to the cladding-coating interface 1259 may not exceed the angle of total internal reflection.

FIG. 17 is a flow chart that illustrates a method for producing a connector portion of a laser-energy delivery device, according to an example. As shown in FIG. 17, a pre-form that has a bore and is made of a doped silica material is received at 1300. The pre-form can be a cylindrical (e.g., tube-shaped) pre-form that has a substantially uniform doping concentration. In some examples, the pre-form can have a non-uniform doping concentration. For example, the pre-form can have a doping concentration that is higher near an inner-surface that defines the bore than at an outer surface of the pre-form, and vice versa. In some examples, the pre-form can have a fluorine doping.

A component is cut from the pre-form at 1310. The component can be cut from the pre-form using, for example, a laser energy cutting instrument or a mechanical cutting instrument. The component can be cut along a plane that is substantially normal to a longitudinal axis (or centerline) of the bore so that the bore is through the entire component. The length of the component can be, for example, a few centimeters.

An inner-surface that defines the bore of the component can be moved over an outer-layer portion of an optical fiber at 1320. Specifically, a distal end of the inner-surface that defines the bore of the component can be moved in a distal direction over a proximal end of the outer-layer portion of the optical fiber. If the size of the bore of the component is defined such that it cannot be moved over the outer-layer portion of the optical fiber (e.g., an inner-diameter of a surface that defines the bore is smaller than an outer diameter of the outer-layer portion of the optical fiber), the size of the bore can be increased using, for example, a reaming process. In some examples, the inner diameter of the surface that defines the bore can be defined so that it is slight larger (e.g., several micrometers larger) than an outer diameter of the outer-layer portion of the optical fiber.

The outer-layer portion of the optical fiber can be associated with, for example, a cladding layer of the optical fiber. The cladding layer can be exposed after a coating and/or a jacket is removed (e.g., stripped) from the cladding layer. In some examples, the outer-layer portion of the optical fiber can be associated with a fiber core of the optical fiber. One more cladding layers can be removed to expose the fiber core of the optical fiber.

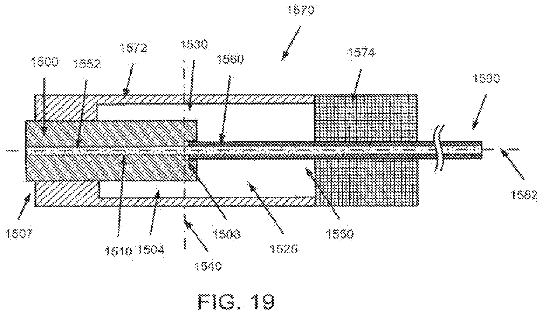

The inner-surface that defines the bore of the component can be moved over the outer-layer portion of the optical fiber until the distal end is within a specified distance of (e.g., within a micrometer, in contact with) an unstripped (e.g., remaining) portion of a jacket, a coating and/or a cladding layer(s) disposed around a portion of the optical fiber. In some examples, the unstripped portion of the jacket, the coating, and/or the cladding layer can be a stop for the component. In some examples, a portion of the jacket, the coating, and/or the cladding layer(s) can be disposed within a portion of the bore of the component (e.g., a tapered portion) after the inner-surface that defines the bore of the component is moved over the outer-layer portion of the optical fiber. A tapered portion of a bore of a component is described in connection with FIGS. 18 and 19.

The inner surface that defines the bore of the component is fused to the outer-layer portion of the optical fiber to produce a connector at 1330. The inner surface can be heat-fused to the outer-layer portion using a heat source such as an electrical heating element, a flame, or a laser energy source (e.g., a carbon dioxide laser energy source). The inner surface can be heat-fused to the outer-layer portion incrementally. The component can be heat-fused to the optical fiber by first heating, for example, a distal end of the component and a distal end of the optical fiber using a heat source until they are heat-fused. The heat source can be moved (e.g., slowly moved) in a proximal direction until the desired portion of the inner surface (e.g., entire inner surface) of the component is heat- fused to the optical fiber. In some embodiments, the component and the optical fiber can be rotated about a longitudinal axis (or centerline) of the optical fiber during the heat-fusing process, for example, to promote even heating and/or heat-fusing around the entire inner surface of the component.

A proximal end of the connector is polished at 1340. The proximal end of the connector (where laser energy can be received) can be polished until the proximal end is substantially flat and substantially normal to a longitudinal axis (or centerline) of the optical fiber. In some examples, the connector can be polished to remove, for example, a portion of a proximal end of the optical fiber protruding from the component. In some examples, the polishing process can include first mechanically grinding the proximal end of the connector. In some examples, the connector can be polished using, for example, a heat source such as a laser energy source.

FIG. 18 is a schematic diagram that illustrates a side cross-sectional view of a doped silica capillary 1400 that has a receiving portion 1407, according to an example. As shown in FIG. 18, the doped silica capillary 1400 has a bore 1410 through an entire length H of the doped silica capillary 1400. In other words, the bore 1410 is in fluid communication with an opening 1420 at a proximal end of the doped silica capillary and an opening 1430 at a distal end of the doped silica capillary 1400. The bore 1410 has a distal portion 1406 that has a diameter J that is greater than a diameter K of a proximal portion 1402 of the bore 1410.

The bore has a tapered portion 1408 disposed between the distal portion 1406 of the bore 1410 and the proximal portion 1402 of the bore 1410. The tapered portion 1408 can taper along a longitudinal axis 1440 (or centerline) of the doped silica capillary 1400 as shown in FIG. 18. In this example, the taper portion 1408 increases in size in a distal direction along the bore 1410. In some examples, the taper 1408 can have flat portions (not shown).

The tapered portion 1408 and the distal portion 1406 of the bore 1410 can collectively be referred to as the receiving portion 1407. Although not shown, in some examples, a proximal end of an optical fiber (not shown) can be inserted into the receiving portion 1407 of the bore 1410 before the doped silica capillary 1400 is heat-fused to the optical fiber. In some examples, a stripped portion of the optical fiber can be inserted into the distal portion 1406 of the bore 1410 at the receiving portion 1407 and then into the remainder of the bore 1410 (e.g., the proximal portion 1402 of the bore 1410). The diameter J of the bore 1410 at the receiving portion 1407 can have a size defined so that an unstripped portion of the optical fiber (e.g., an optical fiber with a jacket, a coating, and/or a cladding layer(s)) can fit into the bore 1410 at the receiving portion 1407. In some examples, the diameter J can be defined based on a diameter of a fiber core, a cladding layer, and/or a coating of an optical fiber configured to be heat-fused to the doped silica capillary 1400. For example, the diameter J can be 5 % to 100 % larger than a diameter of a fiber core, a cladding layer, and/or a coating of an optical fiber.

The receiving portion 1407 can have a length G that is approximately 1 % to 20 % of the entire length H of the doped silica capillary 1400. In some examples, the length G can be between 0.5 mm and 10 mm. In some examples, the length H can be between 100 mm to 10 cm. In some examples, a doped silica capillary 1400 can be defined with an abrupt change between two different sized (e.g., different diameter) lumen that define the bore 1410. In other words, the doped silica capillary 1400 can be defined without a tapered portion 1408.

FIG. 19 is a schematic diagram that illustrates at least a portion of a laser-energy delivery device 1550 disposed within a housing assembly 1570, according to an example. The laser-energy delivery device 1550 has a connector portion 1507 at a proximal portion of the laser-energy delivery device 1550. The laser-energy delivery device 1550 has a portion of an optical fiber 1552 (e.g., an optical fiber core and an optical fiber cladding layer(s)) disposed within a bore 1510 of a doped silica capillary 1500 of the connector portion 1507. Distal to the doped silica capillary 1500, the optical fiber 1552 also has a coating 1560. The coating 1560 can include, for example, an acrylate coating, or an acrylate coating and a polymer-based jacket.

The housing assembly 570 has a capillary holder 572 coupled to the doped silica capillary 500 of the connector portion 507 of the laser-energy delivery device 550. In some examples, the capillary holder 572 can be, for example, mechanically coupled to (e.g., friction fit with, press fit with, mechanically locked to) and/or adhesively coupled to the doped silica capillary 500.

As shown in FIG. 19, the capillary holder 1572 is coupled to a proximal end portion of the doped silica capillary 1500, but need not be coupled to a distal end portion 1504 of the doped silica capillary 1500. In some examples, the capillary holder 1572 can be coupled to a portion of the doped silica capillary 1500 that is distal to a receiving portion 1508. In some examples, the capillary holder 1572 can be coupled to a portion of the doped silica capillary 1500 that is distal to a plane 1540 that is substantially normal to a longitudinal axis 1582 (or centerline) of the laser-energy delivery device 550 and that is at a proximal end of the receiving portion 1508. As shown in FIG. 19, the capillary holder 1572 is coupled to the doped silica capillary 1500 such that an air gap 1525 is disposed between the capillary holder 1572 and the distal end portion 1504 of the doped silica capillary 1500.

The housing assembly 1570 also has an alignment assembly 1574 coupled to the coating 1560 of the optical fiber 1552. In some examples, the alignment assembly 1574 can be, for example, mechanically coupled to (e.g., friction fit with, press fit with, mechanically locked to) and/or adhesively coupled to the coating 1560. The alignment assembly 1574 can be configured hold the optical fiber 1552 so that it substantially does not bend lateral to a longitudinal axis 1582 (or centerline) of the optical fiber 1552. For example, the alignment assembly 1574 can be configured hold the optical fiber 1552 so that it does not substantially bend in a direction substantially normal to a longitudinal axis 1582 (or centerline) of the optical fiber 1552. In some examples, the optical fiber 1552 can hold the optical fiber 1552 without plastically deforming, for example, the coating 1560 or substantially altering the optical characteristics of the optical fiber 1552.

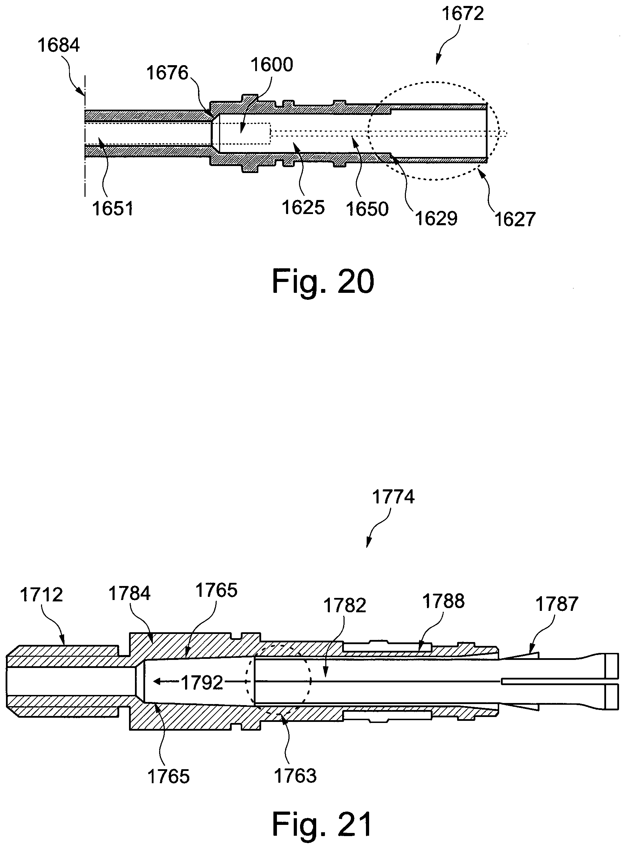

The alignment assembly 1574 can include, for example, a Sub-Miniature A (SMA) connector such as an SMA 905 connector. As shown in FIG. 5, the capillary holder 1572 is coupled to the doped silica capillary 1500 such that an air gap 1525 is disposed between the alignment assembly 1574 and the distal end portion 1504 of the doped silica capillary 1500. In some examples, the capillary holder 1572 can be coupled to the alignment assembly 1574. More details related to capillary holders and alignment assemblies are described in connection with FIGS. 20 through 22B.

As shown in FIG. 19, a portion of the coating 1560 is at least partially disposed within the receiving portion 1508 of the bore 1510 of the doped silica capillary 1500. In some examples, the portion of the coating 1560 can be, for example, adhesively coupled to an inner surface of the receiving portion 1508 of the bore 1510.

FIG. 20 is a schematic diagram of a side cross-sectional view of a capillary holder 1672, according to an example. A doped silica capillary 1600 of a laser-energy delivery device 1650 (shown in dashed lines) is disposed within and coupled to the capillary holder 1672. As shown in FIG. 20, a proximal end 1651 of the laser-energy delivery device 1650 and a proximal end of the capillary holder 1672 are within a plane 1684. The capillary holder 1672 has a taper portion 1676 configured to facilitate ease of insertion of the proximal end 1651 of the doped silica capillary 1600 into the capillary holder 1672 during assembly.

The capillary holder 1672 has a portion 1627 configured to a receive a proximal end of an alignment assembly (not shown). FIG. 21 illustrates an example of an alignment assembly that can be inserted into the portion 1627 of the capillary holder 1672 shown in FIG. 20. Referring back to FIG. 20, the capillary holder 1629 has a stop configured to prevent the alignment assembly from being inserted too far within the capillary holder 1672. In some examples, the capillary holder 1672 can be mechanically coupled to (e.g., press fit with, mechanically locked to, screw fit within) and/or adhesively coupled to the alignment assembly.

FIG. 21 is a schematic diagram of a side cross-sectional view of an alignment assembly 1774, according to an example. As shown in FIG. 21, the alignment assembly 1774 includes a transition component 1784 and an SMA connector component 1782. The transition component 1784 is configured to be coupled to (e.g., lockably coupled to) a capillary holder (not shown) such as that shown in FIG. 20. Specifically, a proximal end 1712 of the transition component 1784 shown in FIG. 21 can be disposed within a capillary holder when coupled to the capillary holder. In some examples, at least a portion of the transition component 1784 can be configured to be disposed outside of a capillary holder when coupled to the capillary holder. The transition component 1784 and SMA connector component 1782 can be moved over a laser-energy delivery device (not shown), for example, disposed within a capillary holder (not shown).

As shown in FIG. 21, the transition component 1784 has a tapered inner wall 1765 and the SMA connector component 1782 has a slotted cylindrical press fit component 1763. The slotted cylindrical press fit component 1763 can also be referred to as a collet 1763. As the collet 1763 is moved in a proximal direction 1792 within the transition component 1784 and moved against the tapered inner wall 1765 of the transition component 1784, the collet 1763 is configured to constrict around and hold a laser-energy delivery device disposed within the SMA connector component 1782. In some embodiments, a connector component (not shown) can be configured to be coupled to at least a portion of a laser-energy delivery device using a different mechanism. For example, the connector component can be configured to clamp around the portion of the laser-energy delivery device via a set screw, a constricting collar (that may be a separately manufactured component), and so forth. The connector component can also be coupled to the portion of the laser-energy delivery device using, for example, an adhesive.

The SMA connector component 1782 is configured to be mechanically coupled to the transition component 1784 via a protrusion 1787 that mechanically locks into a protrusion 1788 of the transition component 1784. As shown in FIG. 21, the SMA connector component 1782 is partially disposed within, but not yet lockably coupled to the transition component 1784. The SMA connector component 1782 can be lockably coupled to the transition component 1784 by moving the SMA connector component 1782 in a proximal direction 1792 within the transition component 1784 until the protrusion 1787 is disposed proximal to the protrusion 1788 of the transition component 1784.

Although the SMA connector component 1782 is configured to be disposed inside of the transition component 1784 (as shown in FIG. 21), in some examples, at least a portion of a connector component (not shown) can be configured to be disposed outside of (e.g., radially outside of) the transition component (not shown). In some examples, the connector component can be made of multiple pieces. In some examples, a connector component can be configured to be coupled to a transition component via a screw mechanism, an adhesive, multiple locking mechanisms, and so forth. In some examples, the connector component can have, for example, threads dispose on an outside portion of the connector component and the transition component can be configured to received the threads of the connector component. When the connector component is screwed into the transition component via the threads, the connector component can be configured to constrict around, for example, at least a portion of a laser-energy delivery device.

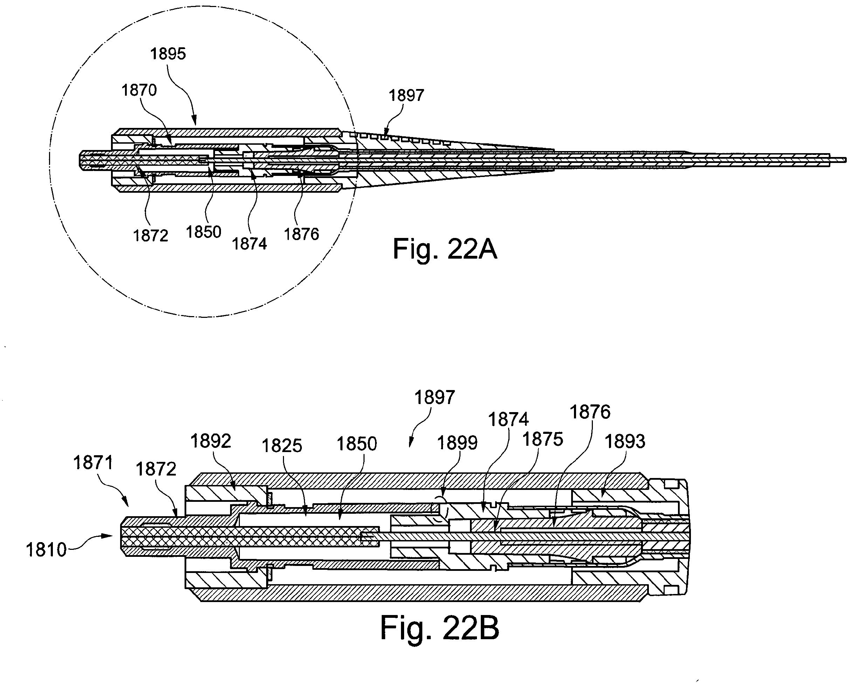

FIG. 22A is a schematic diagram of a side cross-sectional view of a grip assembly 1895, according to an example. A housing assembly 1870 is disposed within the grip assembly 1895, which is coupled to a boot 1897. In some examples, the boot 1897 can be made of a rigid material (e.g., a rigid plastic material), and, in some examples, the boot 1897 can be made of a flexible material (e.g., a flexible rubber material, a flexible plastic material). A laser-energy delivery device 1850 is coupled to a capillary holder 1872, which is coupled to an alignment assembly that includes a transition component 1874 at least partially disposed around an SMA connector component 1876. An enlarged portion M of the grip assembly 1895 is shown in FIG. 22B.

FIG. 22B is a schematic diagram of an enlarged view of the side cross-sectional view of the grip assembly 1895 shown in FIG. 22A, according to an example. Laser energy from, for example, a laser energy source (not shown) can be received at a proximal end 1810 of the laser-energy delivery device 1850. A proximal end portion 1871 of the capillary holder 1872 can be disposed within (e.g., proximate to) the laser energy source.

As shown in FIG. 22B, the capillary holder 1872 is coupled to the grip assembly 1895 via a first coupling nut 1892 and a second coupling nut 1893. The transition component 1874 of the alignment assembly can be coupled to the capillary holder 1872 at 1899 via a locking mechanism (the locking mechanism is not shown). For example, a locking mechanism can include a protrusion from the capillary holder 1872 that can be disposed within a cavity of the transition component 1874. As shown in FIG. 22B, the SMA connector component 1876 is holding the laser-energy delivery device 1850 at 1875.

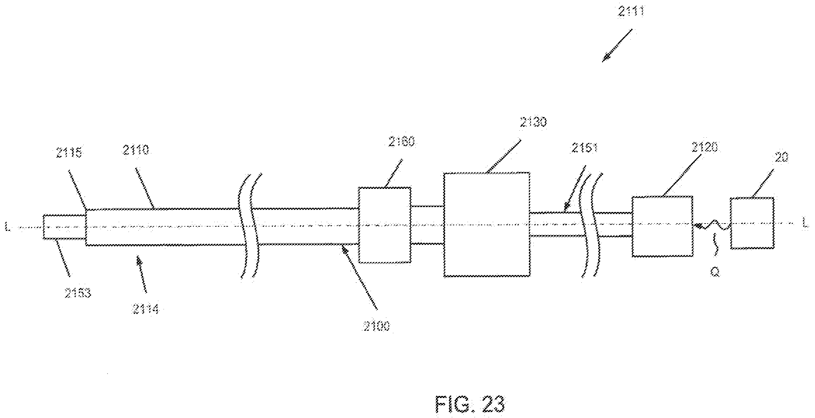

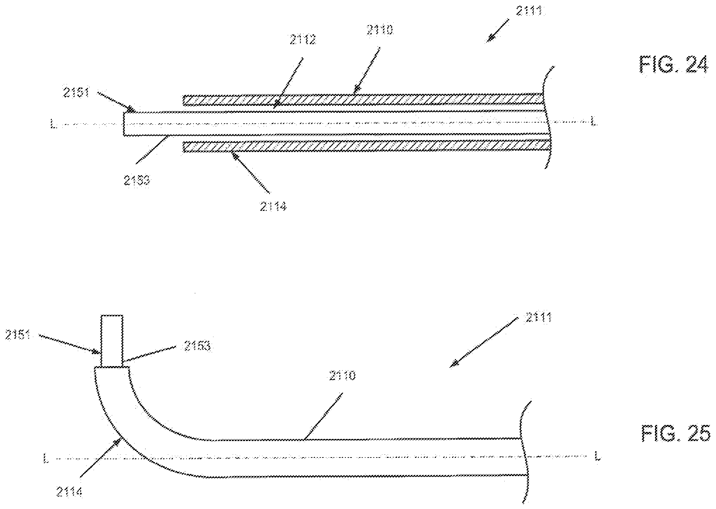

FIGS. 23-28 illustrate a steerable laser-energy delivery device according to one example in which a steerable laser-energy delivery device includes a steerable medical device used in combination with a flexible optical fiber for use in delivering laser energy to a target location within a patient. A steerable laser-energy delivery device 2111 includes a steerable medical device 2100 that includes an elongated member 2110 (also referred to as "sheath" or "tubular member"), a steering mechanism 2130, and an attachment member 2160. In this embodiment, an optical fiber 2151 is slidably or movably disposable within a lumen 2112 (shown in FIG. 24) of the elongated member 2110.

The optical fiber 2151 can be coupled to a connector 2120 configured to receive laser energy Q from a laser energy source 20. The connector 2120 can be, for example, a Stainless Steel SMA 905 standard connector. As discussed above, the laser energy source 20 can have a control module (not shown) configured to control (e.g., set, modify) a timing, a wavelength, and/or a power of the emitted laser energy Q. In some embodiments, the laser energy Q can have a power of between 1 watt and 10 kilowatts. In some embodiments, the control module can also be configured to perform various functions such as laser selection, filtering, temperature compensation, and/or Q-switching. The control module can be a hardware-based control module and/or a software-based control module that can include, for example, a processor and/or a memory.

The steerable medical device 2100 can be constructed the same or similar to, and provide the same or similar functions, as the steerable medical device 200 described above. Thus, the steerable medical device 2100 is not described in detail with reference to this example.

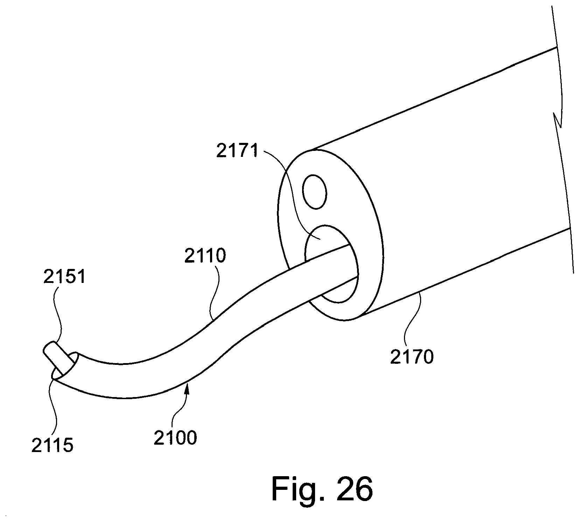

The elongated member 2110 includes a proximal end (no shown) and a distal end 2115, and the lumen 2112 extends between the proximal end and the distal end 2115. A portion of the elongated member 2110 extends through a lumen (not shown) of the attachment member 2160. The elongated member 2110 can be inserted through a working channel 2371 of an endoscope 2370 as shown in FIG. 26. The attachment member 2160 is adapted to couple the device 2100 to the endoscope 2370 as previously described.

As described above, the elongated member 2110 is configured to receive at least a portion of the optical fiber 2151 through the lumen 2112 of the elongated member 2110. For example, the optical fiber 2151 can be inserted into the lumen 2112 at the proximal end of the elongated member 2110. The optical fiber 2151 can be passed through the lumen 2112 of the tubular member 2110 until an advancing end (also referred to as "leading end" or "distal end") of the optical fiber 2151 extends beyond the distal end 2115 of the elongated member 2110 as shown in FIGS. 23-26.

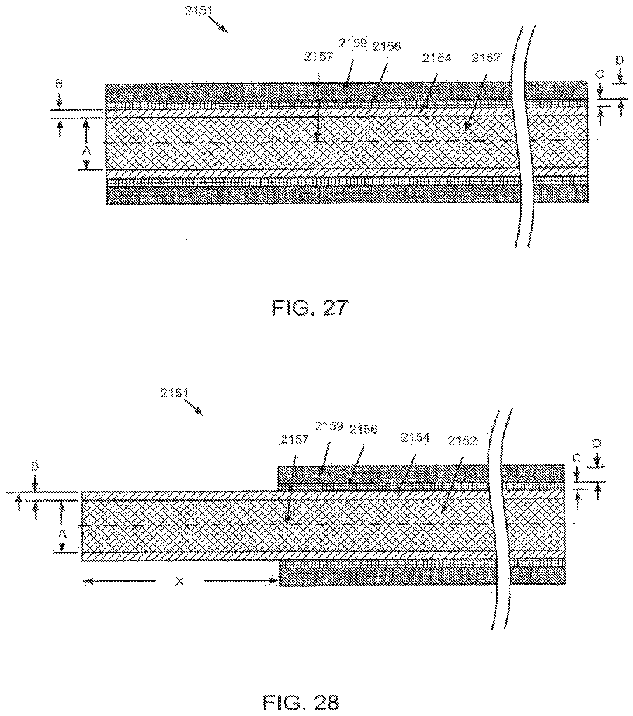

The steering mechanism 2130 is adapted to deflect (e.g., bend, curve or angle) a deflectable portion 2114 of the elongated member 2110 (as shown in FIG. 25), which in turn allows an advancing distal end portion 2153 of the optical fiber 2151 to be controllably directed or guided to a target location. The deflectable portion 2114 of the elongated member 2110 is adapted to be deflected in at least a first direction. The tubular member 2110 can be moved from a linear or straight configuration (or substantially linear or straight configuration) in which at least the deflectable portion 2114 of the elongated member 2110 defines a centerline of longitudinal axis L, as shown in FIGS. 23 and 24, to a deflected configuration in which the deflectable portion 2114 is moved off of (or away from) the longitudinal axis L (e.g., bent, angled or curved), as illustrated in FIG. 25. Thus, as the elongated tubular member 2110 is moved between a linear or straight configuration and a deflected configuration, the portion of the optical fiber 2151 that is disposed within the portion of the lumen 2112 of the elongated member 2110 associated with the deflectable portion 2114, will also be moved between a substantially linear or straight configuration (e.g., and a deflected configuration (bent, angled or curved) in which at least a distal end portion of the optical fiber 2151 is moved off or away from its longitudinal axis or centerline 2157 (shown in FIGS. 27 and 28).

As described above for previous examples of an optical fiber, the optical fiber 2151 can be a silica-based optical fiber and can have, for example, a fiber core 2152 as shown in FIG. 27. In some embodiments, the fiber core 2152 can be made of a suitable material for the transmission of laser energy Q from the laser energy source 20. In some embodiments, for example, the fiber core 2152 an be made of silica with a low hydroxyl (OH ⁻) ion residual concentration. Laser energy can be propagated within the fiber core 2152 during a surgical procedure. The fiber core 2152 can be a multi-mode fiber core and can have a step or graded index profile. The fiber core 2152 can also be doped with a concentration of a dopant (e.g., an amplifying dopant).

The optical fiber 2151 can also have one or more cladding layers 2154 and/or a buffer or coating layer 2156, such as an acrylate layer. The fiber core 2152 and/or cladding layer(s) 2154 can be pure silica and/or doped with, for example, fluorine. The cladding layer(s) 2154 can be, for example, a single or a double cladding that can be made of a hard polymer or silica. The buffer layer 2156 can be made of a hard polymer such as Tefzel®, for example. The optical fiber 2151 can also include a jacket 2159. In such an embodiment, the jacket 2159 can be made of Tefzel®, for example, or can be made of other polymer-based substances. Prior to use, the cladding layer 2154 can be exposed after the buffer layer 2156 and/or the jacket 2159 is removed (e.g., stripped) from the cladding layer 2154. In some embodiments, the one more cladding layers 2154 can be removed to expose the fiber core 2152 prior to use.

The fiber core 2152 of the optical fiber 2151 can have an outer diameter A, for example, between approximately 20 micrometers (µm) to 1200 µm. The cladding layer 2154 of the optical fiber 2151 can have a thickness B, for example, between approximately 5 µm to 120 µm. In some embodiments, the outer diameter (not shown) of the cladding layer 2154 can be 1 to 1.3 times the outer diameter A of the fiber core 2152 of the optical fiber 2151.

The coating or buffer layer 2156 of the optical fiber 2151 can have a thickness C, for example, between approximately 5 µm to 60 µm. The thickness of the coating 2156 of the optical fiber 2151 can be defined to increase the mechanical strength of the optical fiber 2151 during flexing of the optical fiber 2151. The jacket 2159 of the optical fiber 2151 can have a thickness D, for example, between approximately 5 µm to 500 µm.

The optical fiber 2151 can be sized and constructed to allow the optical fiber 2151 to be sufficiently flexible and enable the optical fiber 2151 to be deflected (bent, angled, curved) away from its longitudinal centerline 2157. For example, the fiber core 2152 of the optical fiber 2151 can have a relatively small outer diameter to provide flexibility and reduce the potential for the fiber to be damaged or broken. Although the fiber core 2152 can be constructed with a variety of different outer diameters as described above, a fiber core with an outer diameter, for example, of less than or equal to about 250 microns can improve flexibility to allow the optical fiber to be deflected or steered as described above. For example, in some embodiments, the optical fiber 2151 can include a fiber core 2152 with an outer diameter of about 250 microns. In some embodiments, the fiber core 2152 can have an outer diameter of about 200 microns. In some embodiments, the fiber core 2152 can have an outer diameter of about 240 microns.

The various layers (e.g., cladding, buffer jacket, etc.) of the optical fiber 2151 can add strength to allow the device to receive and deliver relatively high levels of laser energy to a target location. For example, in some examples, the steerable laser-energy delivery device 2111 can be rated to deliver laser energy at up to 100 watts. In addition, the added strength of the elongate tubular member 2110, and the ability to steer the distal end portion of the optical fiber 2151 can improve control of the laser energy. Such control can reduce operating time, improve reliability and durability of the device and reduce cost. Thus, the device is capable of being adjusted from a straight fire (e.g., 0 degrees) to a side fire laser delivery device. In some embodiments, the optical fiber 2151 can be deflected up to, for example, 70 degrees away from its longitudinal axis A. In some embodiments, the optical fiber 2151 can be deflected up to a radius of curvature of, for example, 1 cm.

In some embodiments, the distal portion of various layers (e.g., a buffer layer and/or a jacket and/or a cladding layer) that is typically stripped from the optical fiber 2151 to expose the fiber core and/or the cladding layer prior to delivering the laser energy can extend, for example, a distance X, as shown in FIG. 28. FIG. 28 illustrates the jacket 2159 and the buffer or coating layer 2156 stripped back, but it should be understood that in some embodiments, only the jacket is stripped. The length of the stripped portion of the optical fiber can vary depending on the particular need. For example, in some embodiments, the distance X can be for example up to about 10 cm from the distal end of the optical fiber 2151. In some embodiments, the distance X can be, for example, between about 1 mm and 10 mm. In some embodiments, the distance X can be about 3 cm. A larger distance X allows for more of the optical fiber 2151 to be extended outside of the lumen of the elongated member 2110 as needed or desired.

As discussed above, the optical fiber 2151 can be slidably received within the lumen of the elongated member 2110, which allows the optical fiber 2151 to be moved distally outside the distal end of the elongated member 2110, incrementally or continuously, as needed, during a medical procedure. For example, in use, the distal end portion 2153 of the optical fiber 2151 can be extended distally out of the lumen 2112 of the elongated member 2110 a sufficient distance to allow the optical fiber 2151 to deliver laser energy to a target location within a patient. If a distal tip portion of the optical fiber 2151 is subsequently burned (commonly referred to as "burn-back") during the procedure, the optical fiber 2151 can be further extended outside the lumen 2112 of the elongated member 2110 to allow for additional or continual laser energy to be applied.

In alternative examples, a steerable laser-energy delivery device can include an optical fiber constructed the same or similar to the optical fiber 1150, the optical fiber 1251 or the optical fiber 1552 described herein. In such examples, rather than a connector 2120, the steerable laser delivery device can optionally include a connector portion constructed the same, or similar to, for example, the connector portion 1120, the connector portion 1225, or the connector portion 1507 described herein. In some examples, a steerable laser-energy delivery device may not include an attachment member 2160.

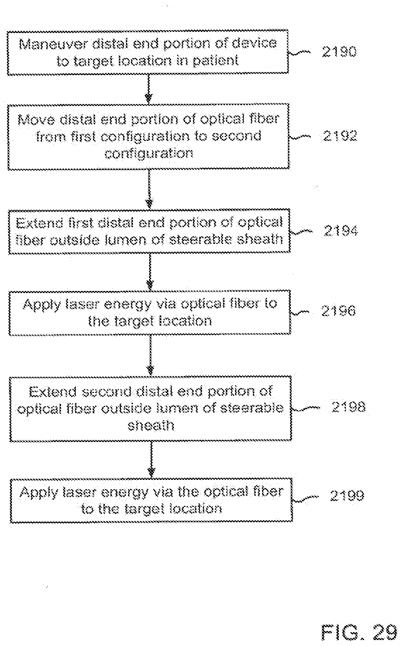

FIG. 29 is a flowchart illustrating one example method of using the laser-energy delivery device 2110. At 2190 a distal end portion of a steerable laser-energy delivery device is maneuvered or steered to a target location within a patient's body while the steerable laser-energy delivery device is in a substantially linear configuration. The steerable laser-energy delivery device includes at least a portion of an optical fiber movably disposed within a lumen of a steerable sheath. As discussed above, prior to maneuvering the steerable laser-energy delivery device to a target location, a portion of an outer layer (e.g., the jacket) of the optical fiber can be removed from the distal end portion of the optical fiber. For example, a portion of the outer layer (e.g., jacket) between 1 mm and 10 cm from a distal end of the optical fiber can be removed. In addition, as described above, in some examples, prior to maneuvering the steerable laser-energy delivery device to a target location, at least a portion of the steerable laser-energy delivery device can optionally be inserted through a lumen of an endoscope.

At 2192, the distal end portion of the steerable laser-energy delivery device is moved from a first configuration in which the distal end portion of the optical fiber is substantially linear and defines a longitudinal axis, to a second configuration in which the distal end portion of the optical fiber is moved off its longitudinal axis. In some examples, the distal end portion of the optical fiber is configured to be deflected up to a bend radius of about 1 cm. In some embodiments, the distal end portion of the optical fiber is configured to be deflected up to 70 degrees relative to its longitudinal axis.

At 2194, a first distal end portion of the optical fiber is extended outside the lumen of the steerable sheath at a distal end of the steerable sheath. At 2196, after extending the first distal end portion of the optical fiber, laser energy is applied via the optical fiber to the target location within the patient. For example, in some examples, laser energy up to 100 Watts of power can be applied.

At 2198, after applying the laser energy, the distal end of the optical fiber can optionally be extended again outside the lumen of the steerable sheath at a distal end of the steerable sheath. For example, as described above, if the distal end of the optical fiber is burned off during the procedure, it may be desirable to extend an additional length (e.g., a second distal end portion) of the optical fiber outside of the lumen of the steerable sheath. Laser energy can then be applied again to a target location, at 2199.

FIGS. 30 and 31 illustrate an embodiment of a steerable laser-energy delivery device in accordance with the invention that includes a steering mechanism. A steerable laser-energy delivery device 2211 includes a sheath 2210, an optical fiber 2251, an outer tubular member 2261, and a connector (not shown) configured to receive laser energy from a laser energy source (not shown). The outer tubular member 2261 can be, for example, a portion of an endoscope or other similar type of medical instrument. The optical fiber 2251 can be constructed, for example, the same or similar to the optical fiber 2151. The connector and the laser energy source can also be, for example, the same as the connector 2120 and laser energy source 20 previously described and are therefore not described in detail below.

In this embodiment, the sheath 2210 is formed with a shape-memory material, such as Nitinol, such that it can be biased into a desired shape. For example, a distal end portion 2214 of the sheath 2210 can be formed to have a biased curved or angled configuration. The optical fiber 2251 can be disposed within a lumen 2212 of the sheath 2210, and the sheath 2210 can be slidably received within a lumen 2263 of the outer tubular member 2261. In some embodiments, the sheath 2212 can be fixed to the optical fiber 2251, for example, with adhesives or other attachment methods. In some embodiments, the optical fiber 2251 can be slidably received within the lumen 2212 of the sheath 2210.

When the distal end portion 2214 of the sheath 2210 is disposed within the lumen 2263 of the outer tubular member 2261, the sheath 2210 will be restrained and maintained in a substantially linear or straight configuration, as shown in FIG. 30. When the distal end portion 2214 of the sheath 2210 is disposed outside the lumen 2263 of the outer tubular member 2261 at a distal end 2265 of the outer tubular member 2261 (i.e., the sheath 2210 is unrestrained), the sheath 2210 will be free to assume its biased configuration, as shown in FIG. 31. For example, in some embodiments, the sheath 2210 can be moved distally relative to the outer tubular member 2261. In some embodiments, the outer tubular member 2261 is moved proximally relative to the sheath 2210. In either case, the unrestrained distal end portion of the sheath 2210 will be free to move to its biased configuration, and the optical fiber 2251 will also be moved from a substantially linear or straight configuration to a deflected configuration (e.g., away from a longitudinal axis A defined by the optical fiber 2251), as shown in FIG. 31.

While various embodiments have been described above, it should be understood that they have been presented by way of example only, and not limitation. For example, a steerable laser-energy delivery device can include various combinations and/or sub-combinations of the various components and/or features described herein. In addition, other types of steering mechanisms can be used in conjunction with the various embodiments of an optical fiber and/or a laser-energy delivery device as described herein. For example, other types of steerable sheaths or cannulas can be used with an optical fiber or laser-energy delivery device as described herein. Similarly, various types and embodiments of optical fibers not described herein can be used in conjunction with a steering mechanism or steerable medical device described herein.

In another example, the optical fiber components (e.g., connector end portion, laser-energy-delivery device, grip assembly) described herein can include various combinations and/or sub-combinations of the components and/or features of the different embodiments described. The optical fiber components, as well as the methods of using the optical fiber components, can be used in the treatment of various conditions in addition to those mentioned herein.

## Claims

1. An apparatus comprising:
an optical fiber (2251) configured to deliver laser energy to a target area within a patient, the optical fiber (2251) being sufficiently flexible such that the optical fiber (2251) can be moved from a first configuration in which a distal end portion of the optical fiber (2151) is substantially linear and defines a longitudinal axis (L) to a second configuration in which the distal end portion of the optical fiber (2251) is deflected off its longitudinal axis (L) ;
a sheath (2210) coupled to the optical fiber (2251) and configured to move the optical fiber (2151) between its first configuration and its second configuration, the optical fiber (2251) being disposed within a lumen (2212) of the sheath (2210) being formed with a shape-memory material and having a distal end portion (2214) biased into a curved configuration; and
an outer tubular member (2261) comprising a lumen (2263) for slidably receiving the sheath (2210), wherein, when the distal end portion (2214) of the sheath (2210) is disposed within the lumen (2263) of the outer tubular member (2261), the sheath (2210) is maintained in a linear configuration, and when the distal end portion (2214) of the sheath (2210) is disposed outside the lumen (2263) of the outer tubular member (2261), the sheath (2210) assumes its biased curved configuration, such that the optical fiber (2251) is moved from its first configuration to its second configuration.

2. The apparatus of claim 1, wherein the outer tubular member (2261) is a portion of an endoscope.

3. The apparatus of claim 1, wherein the sheath (2212) is fixed to the optical fiber (2251).

4. The apparatus of claim 1, wherein the distal end portion of the optical fiber (2251) is configured to be deflected up to 70 degrees relative to its longitudinal axis (L) when in its second configuration.

5. The apparatus of claim 1, wherein the distal end portion of the optical fiber (2251) is configured to be deflected up to a bend radius of about 1 cm when in its second configuration.

6. The apparatus of claim 1, wherein the optical fiber (2251) includes a fiber core with an outer diameter of less than or equal to 250 microns.

7. The apparatus of claim 1, wherein the optical fiber (2251) includes a fiber core with an outer diameter less than or equal to 200 microns.

8. The apparatus of claim 1, wherein a distal end of the optical fiber (2251) has a larger diameter than a remaining portion of the optical fiber (2251).

9. The apparatus of claim 1, wherein the optical fiber (2251) is configured to deliver laser energy at up to at least 100 watts of power.

10. The apparatus of claim 1, **characterized by**:
a steering mechanism (2130) adapted to deflect the distal end (2115) of the steerable sheath (2110), wherein the steering mechanism (2130) comprises a housing (240) and an actuator (244), and the housing (240) and the steerable sheath (2110) are fixedly coupled, and wherein the actuator (244) is movable with respect to the housing (240) such that this movement controls the deflection of the distal end (2115) of the steerable sheath (2110).

## Patentansprüche

1. Vorrichtung, umfassend:
eine optische Faser (2251), die eingerichtet ist, Laserenergie an einen Zielbereich innerhalb eines Patienten zu liefern, wobei die optische Faser (2251) ausreichend flexibel ist, derart dass die optische Faser (2251) von einer ersten Konfiguration, in der ein distaler Endabschnitt der optischen Faser (2151) im Wesentlichen linear ist und eine Längsachse (L) definiert, zu einer zweiten Konfiguration bewegt werden kann, in der der distale Endabschnitt der optischen Faser (2251) von seiner Längsachse (L) abgelenkt ist;
eine Hülle (2210), die mit der optischen Faser (2251) gekoppelt und eingerichtet ist, die optische Faser (2151) zwischen ihrer ersten Konfiguration und ihrer zweiten Konfiguration zu bewegen, wobei die optische Faser (2251) innerhalb eines Lumens (2212) der Hülle (2210) angeordnet ist, die mit einem Formgedächtnismaterial ausgebildet ist und einen distalen Endabschnitt (2214) aufweist, der in eine gebogene Konfiguration vorgespannt ist; und ein äußeres rohrförmiges Element (2261), ein Lumen (2263) umfassend, zum gleitenden Aufnehmen der Hülle (2210), wobei, wenn der distale Endabschnitt (2214) der Hülle (2210) innerhalb des Lumens (2263) des äußeren rohrförmigen Elements (2261) angeordnet ist, die Hülle (2210) in einer linearen Konfiguration gehalten wird, und wenn der distale Endabschnitt (2214) der Hülle (2210) außerhalb des Lumens (2263) des äußeren rohrförmigen Elements (2261) angeordnet ist, die Hülle (2210) ihre vorgespannte gekrümmte Konfiguration annimmt, so dass die optische Faser (2251) von ihrer ersten Konfiguration zu ihrer zweiten Konfiguration bewegt wird.

2. Vorrichtung nach Anspruch 1, wobei das äußere rohrförmige Element (2261) ein Abschnitt eines Endoskops ist.

3. Vorrichtung nach Anspruch 1, wobei die Hülle (2212) an der optischen Faser (2251) befestigt ist.

4. Vorrichtung nach Anspruch 1, wobei der distale Endabschnitt der optischen Faser (2251) eingerichtet ist, bis zu 70 Grad in Bezug auf seine Längsachse (L) abgelenkt zu werden, wenn er sich in seiner zweiten Konfiguration befindet.

5. Vorrichtung nach Anspruch 1, wobei der distale Endabschnitt der optischen Faser (2251) eingerichtet ist, bis zu einem Biegeradius von etwa 1 cm abgelenkt zu werden, wenn er sich in seiner zweiten Konfiguration befindet.

6. Vorrichtung nach Anspruch 1, wobei die optische Faser (2251) einen Faserkern mit einem äußeren Durchmesser von weniger als oder gleich 250 Mikrometer umfasst.

7. Vorrichtung nach Anspruch 1, wobei die optische Faser (2251) einen Faserkern mit einem äußeren Durchmesser weniger als oder gleich 200 Mikrometer umfasst.

8. Vorrichtung nach Anspruch 1, wobei ein distales Ende der optischen Faser (2251) einen größeren Durchmesser aufweist als ein verbleibender Abschnitt der optischen Faser (2251).

9. Vorrichtung nach Anspruch 1, wobei die optische Faser (2251) eingerichtet ist, Laserenergie mit bis zu mindestens 100 Watt Leistung zu liefern.

10. Vorrichtung nach Anspruch 1, **gekennzeichnet durch**: einen Lenkmechanismus (2130), der angepasst ist, das distale Ende (2115) der lenkbaren Hülle (2110) abzulenken, wobei der Lenkmechanismus (2130) ein Gehäuse (240) und ein Stellglied (244) umfasst, und das Gehäuse (240) und die lenkbaren Hülle (2110) fest gekoppelt sind, und wobei das Stellglied (244) in Bezug auf das Gehäuse (240) beweglich ist, sodass diese Bewegung die Ablenkung des distalen Endes (2115) der lenkbaren Hülle (2110) steuert.

## Revendications

1. Appareil comprenant :
une fibre optique (2251) qui est configurée de manière à ce qu'elle délivre de l'énergie laser sur une zone cible sur un patient, la fibre optique (2251) étant suffisamment flexible de telle sorte que la fibre optique (2251) puisse être déplacée depuis une première configuration dans laquelle une partie d'extrémité distale de la fibre optique (2151) est sensiblement linéaire et définit un axe longitudinal (L) jusqu'à une seconde configuration dans laquelle la partie d'extrémité distale de la fibre optique (2251) est déviée par rapport à son axe longitudinal (L) ;
une gaine (2210) qui est couplée à la fibre optique (2251) et qui est configurée de manière à ce qu'elle déplace la fibre optique (2151) entre sa première configuration et sa seconde configuration, la fibre optique (2251) étant disposée à l'intérieur d'une lumière (2212) de la gaine (2210) qui est formée à partir d'un matériau à mémoire de forme et qui comporte une partie d'extrémité distale (2214) qui est sollicitée par poussée selon une configuration incurvée ; et
un élément tubulaire externe (2261) qui comprend une lumière (2263) pour recevoir de façon coulissante la gaine (2210), dans lequel, lorsque la partie d'extrémité distale (2214) de la gaine (2210) est disposée à l'intérieur de la lumière (2263) de l'élément tubulaire externe (2261), la gaine (2210) est maintenue selon une configuration linéaire, et lorsque la partie d'extrémité distale (2214) de la gaine (2210) est disposée à l'extérieur de la lumière (2263) de l'élément tubulaire externe (2261), la gaine (2210) prend sa configuration incurvée sollicitée par poussée, de telle sorte que la fibre optique (2251) soit déplacée depuis sa première configuration jusqu'à sa seconde configuration.

2. Appareil selon la revendication 1, dans lequel l'élément tubulaire externe (2261) est une partie d'un endoscope.

3. Appareil selon la revendication 1, dans lequel la gaine (2212) est fixée à la fibre optique (2251).

4. Appareil selon la revendication 1, dans lequel la partie d'extrémité distale de la fibre optique (2251) est configurée de manière à ce qu'elle soit déviée jusqu'à 70 degrés par rapport à son axe longitudinal (L) lorsqu'elle est dans sa seconde configuration.

5. Appareil selon la revendication 1, dans lequel la partie d'extrémité distale de la fibre optique (2251) est configurée de manière à ce qu'elle soit déviée jusqu'à un rayon de courbure d'environ 1 cm lorsqu'elle est dans sa seconde configuration.

6. Appareil selon la revendication 1, dans lequel la fibre optique (2251) inclut une âme de fibre qui présente un diamètre externe inférieur ou égal à 250 micromètres.

7. Appareil selon la revendication 1, dans lequel la fibre optique (2251) inclut une âme de fibre qui présente un diamètre externe inférieur ou égal à 200 micromètres.

8. Appareil selon la revendication 1, dans lequel une extrémité distale de la fibre optique (2251) présente un diamètre qui est plus grand que celui d'une partie restante de la fibre optique (2251).

9. Appareil selon la revendication 1, dans lequel la fibre optique (2251) est configurée de manière à ce qu'elle délivre de l'énergie laser à jusqu'à au moins 100 Watts de puissance.

10. Appareil selon la revendication 1, **caractérisé par** :
un mécanisme d'orientation et de direction (2130) qui est adapté de manière à ce qu'il dévie l'extrémité distale (2115) de la gaine pouvant être orientée et dirigée (2110), dans lequel le mécanisme d'orientation et de direction (2130) comprend un carter (240) et un actionneur (244), et le carter (240) et la gaine pouvant être orientée et dirigée (2110) sont couplés de façon fixe, et dans lequel l'actionneur (244) peut être déplacé par rapport au carter (240) de telle sorte que ce déplacement commande la déviation de l'extrémité distale (2115) de la gaine pouvant être orientée et dirigée (2110).
